# EUROPEAN PATENT APPLICATION

(11) **EP 1 043 588 A1**
(43) Date of publication of application: **11.10.2000**
(21) Application number: 98961590.1
(22) Date of filing: 25.12.1998
(51) Int. Cl.: G01N 33/543, B01D 35/02

(54) **ANALYZER FOR LIQUID SAMPLES**

(30) Priority: 25.12.1997 JP 35862997
(71) Applicant: MOCHIDA PHARMACEUTICAL CO., LTD., Shinjuku-ku Tokyo 160-8515 (JP)
(72) Inventor: TERASAWA, Hideyuki, Mochida Pharmaceutical Co. Ltd, Tokyo 160-8515 (JP); YAMAUCHI, Tadakazu, Mochida Pharmaceutical Co. Ltd, Tokyo 160-8515 (JP)
(74) Representative: Grünecker, Kinkeldey, Stockmair & Schwanhäusser Anwaltssozietät
(86) International application number: JP9805946
(87) International publication number: WO9934213

(57) **Abstract**

An analyzer for liquid samples, comprising a liquid sample introduction member, a filter member for capturing a solid component of a liquid sample introduced from the liquid sample introduction member, and an analyzer member for analyzing an object substance in the liquid sample passed through the filter, wherein a clearance-carrying flow passage securing member is formed so that the permeation of a liquid component in the liquid sample is not prevented by the solid component captured by and accumulated on the filter member between a member, which is provided above the filter member, and the filter member; a specific binding analysis method and a specific binding analysis apparatus.

## Description

### TECHNICAL FIELD

The present invention relates to an analyzer for liquid samples, to a specific binding analysis apparatus and to a specific binding analysis method. More particularly, it relates to a liquid sample analysis apparatus for analyzing a liquid sample possibly containing a solid component such as a blood component, a tissue component, or dust, the apparatus being capable of accurately and efficiently analyzing an object substance in the liquid sample in the analysis member provided downstream of a filter member such that the permeation of a liquid component of the liquid sample is not prevented by the solid component captured by the filter member, to specific binding analysis method and apparatus using such a liquid sample analysis apparatus.

### BACKGROUND ART

When analysis is made of a component contained in a liquid sample as a liquid component, a solid component in the liquid sample, which will be a factor that prevents the analysis or inhibits efficient analysis, is preliminarily captured and removed by a filter or the like and thereafter only a liquid component passed through the filter or the like is subjected to the analysis. For example, as an analysis apparatus for analyzing a whole blood sample, there have been known methods and apparatuses in which liquid permeating materials such as glass fiber mass and nonwoven fabric as filters for capturing blood cells (Published Examined Japanese Patent Application No. Hei 6-64054, Published Unexamined Japanese Patent Application No. Hei 8-54387, Published Unexamined Japanese Patent Application No. Hei 3-131757, Published Unexamined Japanese Patent Application No. Hei 3-131759, and Published Unexamined Japanese Patent Application No. Hei 3-163361). However, these methods and apparatuses have the defect that the blood component captured by the filter prevents the permeation of the liquid component to decrease the degree of accuracy in the analysis of an object substance in the liquid component and also prevents quick analysis. This is the problem caused by the fact that generally liquid samples tend to pass through in a shortest distance in a region where it can permeate, such as a filter, with the result that the solid component in the liquid sample is apt to be captured by a local region of the filter, so that in a conventional apparatus, the captured solid component will locally fill the filter or be locally accumulated on the filter according as the liquid sample passes through the filter to prevent local flow of the liquid sample.

Accordingly, there have been proposed the analyzer element having laminated a blood cell separation layer composed of two or more kinds of microporous membranes having different pore size (Published Unexamined Japanese Patent Application No. Hei 5-264539) and the analyzer element having a porous development layer (Published Unexamined Japanese Patent Application No. Hei 5-26875). However, these analyzer elements could have only insufficiently dissolved the problem that the permeation of liquid component is prevented by the blood component captured by the blood cell separation layer or porous development layer, as the liquid sample permeates in a shortest distance.

Further, to improve the ability of capturing blood cells, there have also been proposed the method or apparatus for removing a blood component from a whole blood sample in which an absorbing material impregnated with a blood cell capturing substance such as lectin as an agglutinin is used in the filter member (Published Unexamined Japanese Patent Application No. Sho 61-118661 and Published Unexamined Japanese Patent Application No. Sho 61-207966) and also the apparatus or method in which use is made of an erythrocyte binding component such as lectin and a stabilizer component for agglutinated erythrocytes, such as a polycationic polymer component (Published Unexamined Japanese Patent Application No. Sho 61-118661 and Published Unexamined Japanese Patent Application No. Hei 7-5173). However, even with these apparatuses or methods, the problem of the prevention of permeation of liquid component by the captured blood cell component remains.

As described above, the conventional apparatuses or methods suffer adverse influences that the defect of prevention of permeation of liquid component by the captured blood cell component makes the analysis in the analysis member impossible or inaccurate and that the performance of the analysis apparatus and the results of analysis depend greatly on the amount of solid component (e.g., hematocrit value) occupying the liquid sample.

In the meantime, with the propagation of tests upon medical practice, tests before medical practice, home tests, etc. and an increase in clinical tests, etc. that are highly exigent, there has arisen a keen desire for the development of an analysis apparatus that can analyze whole blood as it is without performing plasma separation or serum separation using a device such as a centrifuge, in particular, an analysis apparatus that enables quick, simple and easy, and reliable analysis and measurement of blood by a person other than an expert of clinical tests.

Accordingly, as an analysis method and apparatus that can meet such a desire, the present inventors have previously developed and proposed an analysis method called MEDIA (Mediator Diffusion-controlled Immunoassay) method which forms a distribution of distance of a signal substance generator corresponding to the amount of the object substance from the detection member utilizing specific binding reaction of an object substance for analysis in the liquid sample and detects by the detection member the distribution as a signal intensity controlled by the distance of the mass transfer of the signal substance that the signal substance generator generated, i.e., diffusion distance (Published Unexamined Japanese Patent Application No. Hei 5-264552, Published Unexamined Japanese Patent Application No. Hei 8-75748, and Published Unexamined Japanese Patent Application No. Hei 9-234097). This method enables simple and easy and quick measurement of the object substance in a liquid sample at high sensitivity without the operation of removal of unreacted substances.

However, even with this analysis method, like in other homogeneous methods, hindrance by the solid component such as blood cell in a whole blood sample would affect the signal intensity at the detection member, giving an error in the results of measurement of the amount of the object substance in the sample.

### SUMMARY OF THE INVENTION

Accordingly, an object of the present invention is to provide an analysis apparatus for a liquid sample, in which upon analysis of liquid component in a liquid sample possibly containing a solid component, the permeation of the liquid component in the liquid sample in a filter member provided for preliminarily capturing the solid component is not prevented by the captured solid component, so that the degree of accuracy in the analysis of the object substance in the liquid component in an analysis member downstream of the filter member can improved, the solid component can be efficiently captured on the filter member to achieve quick analysis, and the whole apparatus including the filter member can be downsized.

Further, another object of the present invention is to provide a specific binding analysis method and a specific binding analysis apparatus that measure an object substance in a liquid sample by its specific binding reaction.

To achieve the above objects, the present invention provides an analyzer for liquid samples, comprising a liquid sample introduction member, a filter member for capturing a solid component of a liquid sample introduced from the liquid sample introduction member, and an analysis member for analyzing an object substance in the liquid sample passed through the filter, wherein a clearance-carrying flow passage securing member is formed so that the permeation of a liquid component in the liquid sample is not prevented by the solid component captured by and accumulated on the filter member between a member, which is provided above the filter member, and the filter member.

Also, provided is the apparatus in which the above flow passage securing member is constituted by a clearance formed between a depression formed on a lower surface of a member formed on a body of the apparatus arranged above the filter member and an upper surface of the filter member.

Further, provided is the apparatus in which the above flow passage securing member is constituted by an insertion member having a liquid passage, inserted between the member arranged above the filter member and the filter member.

Furthermore, the present invention provides a specific binding analysis method and a specific binding analysis apparatus that measure an object substance in a liquid sample by its specific binding reaction using the above analysis apparatus for liquid samples.

### BRIEF DESCRIPTION OF THE DRAWINGS

Fig. 1A is a schematic diagram showing a construction example of the specific binding analysis apparatus of the present invention and Fig. 1B is a cross-sectional view showing it in its assembled state.
Fig. 2A is a cross-sectional view showing a structure of an upper cover of the specific binding analysis apparatus of the present invention and Fig. 2B is its bottom view.
Fig. 3A is a cross-sectional view showing a structure of an upper cover of the specific binding analysis apparatus of the present invention, Fig. 3B is its bottom view, and Fig. 3C is its side view.
Fig. 4A is a cross-sectional view showing another structure of an upper cover of the specific binding analysis apparatus of the present invention and Fig. 4B is its bottom view.
Fig. 5A is a cross-sectional view showing still another structure of an upper cover of the specific binding analysis apparatus of the present invention and Fig. 5B is its bottom view.
Fig. 6A is a cross-sectional view showing yet another structure of an upper cover of the specific binding analysis apparatus of the present invention and Fig. 6B is its bottom view.
Fig. 7A is a cross-sectional view showing another structure of an upper cover of the specific binding analysis apparatus of the present invention and Fig. 7B is its bottom view.
Figs. 8A to D are bottom views showing other structures, respectively, of an upper cover of the specific binding analysis apparatus of the present invention.
Fig. 9A is a cross-sectional view showing another structure of an upper cover of the specific binding analysis apparatus of the present invention and Fig. 9B is its bottom view.
Fig. 10A is a cross-sectional view showing the structure of a lower substrate of the specific binding analysis apparatus of the present invention, Fig. 10B is its bottom view, and Fig. 10C is its side view.
Fig. 11A is a schematic diagram showing another construction example of the specific binding analysis apparatus of the present invention and Fig. 11B is a cross-sectional view showing it in its assembled state.
Figs. 12A to I are views showing other construction examples, respectively, of the liquid non-permeating member of the specific binding analysis apparatus of the present invention shown in Fig. 10.
Fig. 13 is a schematic diagram showing another construction example of the specific binding analysis apparatus of the present invention.
Figs. 14A to D are schematic cross-sectional views showing another construction examples, respectively, of the flow passage securing member of the specific binding analysis apparatus of the present invention.
Figs. 15A to C are views showing construction examples of the specific binding analysis apparatus of the present invention having the flow passage securing member shown in Fig. 14D.
Fig. 16 is a diagram showing the results of Example 1.
Fig. 17 is a diagram showing the results of Example 2.
Figs. 18A and B are diagrams showing the results of Example 3, respectively.
Fig. 19 is a diagram showing the results of Example 4.
Fig. 20 is a diagram showing the results of Example 5.
Figs. 21A and B are diagrams showing the results of Example 6, respectively.
Figs. 22A and B are diagrams showing the results of Example 7, respectively.
Fig. 23 is a diagram showing the results of Example 8.

### Explanation of reference numerals

1 upper cover, 2 filter member,
3 analysis member, 4 lower substrate
11 liquid sample introduction inlet, 12 holding member for a liquid introduction inlet,
13 bottom, 14a, 14b, 14c protrusion
15a, 15b, 15c depression, 21a, 21b glass fiber filter paper
22 liquid non-permeating sealing member, 31 communication hole,
32 electrode substrate, 33 antibody-insolubilized porous membrane,
34 dialysis membrane sealing member, 35 water absorption member (glass fiber filter paper),
36 communication member (communication hole or communication edge),
37 working electrode (detection member),
38 terminal for working electrode,
39 counter electrode,
41 terminal for counter electrode, 42 base,
43a, 43b, 43c, 43d assembly hole,
44a, 44b, 44c, 44d assembly hole,
45a, 45b flow inlet, 46 liquid non-permeating member
51 upper cover, 52 filter member
53 analysis member, 54 lower substrate,
55 insertion element butting member, 56 insertion element holding member,
57 insertion element, 58a, 58b, 58c liquid flow inlet,
59a lower sheet element, 59b upper sheet element,
60 spacer, 61 thin layer flow passage,
62a, 62b nonwoven fabric, 63 filter medium
72 frame, 73 groove,
81 lower substrate, 82 depression
83 clogging-preventing filter, 84 blood cell capturing filter,
84a label impregnation member, 84b enzyme substrate impregnation member,
85 label, 86 non-carried dried reagent,
87 enzyme substrate impregnation member

### BEST MODE FOR CARRYING OUT THE INVENTION

Hereinafter, the liquid sample analysis apparatus of the present invention (hereafter, referred to as "the apparatus of the invention") and specific binding analysis method and specific binding analysis apparatus will be explained in detail.

The apparatus of the invention has a liquid sample introduction member, a filter member for capturing a solid component in the liquid sample introduced from the liquid sample introduction member, and an analysis member for analyzing an object substance in the liquid sample that permeated the filter member.

In the present invention, the liquid sample introduced from the liquid sample introduction member is a liquid that is expected to contain an object substance whose physical properties, chemical composition or content of a specified substance, etc. are to be analyzed or measured. The liquid sample specifically includes urine, serum, plasma, whole blood, saliva, eyedrop, cerebrospinal fluid, or secretions from body organs such as nipple. It may also be solid or gel-like or sol-like substance such as mucus, tissues or cells, bacterial cells, etc. and even suspensions or solutions of these in buffers, extraction liquids, dissolution liquids, etc.

The solid component contained in the liquid sample and captured by the filter member is component that does not participate in the analysis of or can prevent the analysis of a blood cell component, a cell component, a tissue component, an artificial fine particle (latex, polystyrene microspheres, etc.), dust, etc., or their fragments, decomposates, etc. in the analysis member and that can be substantially captured by the filter member.

The liquid component of the liquid sample is a residual liquid component obtained after substantially removing the above solid component, which is an object fraction for analysis.

Further, in the present invention, the object substance is a substance that is measured in the apparatus of the present invention. Specific examples thereof include metabolites, enzyme substrates, enzyme inhibitors, enzyme activators, enzyme molecules, or various proteins functioning as antibody molecules or antigens, polypeptides, glycoproteins, polysaccharides, complex glycolipids, or nucleic acids, effector molecules, receptor molecules, etc.

More specifically, examples thereof include enzyme substrate or metabolites such as creatinine, uric acid, glucose, cholesterol, and neutral fats, acute phase proteins such as C-reactive protein (CRP), tumor markers such as α-fetoprotein, carcinoembryonic antigen (CEA), CA125, CA19-9, various proteins such as β2-microglobulin (β2m), and ferritin; various hormones such as estradiol (E2), estriol (E3), human chorionic gonadotropin (hCG), luteinizing hormone (LH), and human placenta lactogen (hPL); various virus-related antigens or virus-related antibodies such as HBs antigen, HBs antibody, HBe antigen, HBe antibody, HBc antibody, HCV antibody, and HIV antibody; various allergen and IgE antibodies specific thereto; narcotics, medical chemicals and metabolites thereof; nucleic acids of virus and disease-related polynucleotide sequences, etc.

In the apparatus of the invention, the liquid sample introduction member is an inlet for introducing the liquid sample into the apparatus and may be constructed by a microtubular flow passage communicated with the filter member in the apparatus, an opening communicated with a thin layer passage, or an opening formed on the upper part of the body of apparatus. Of these, the one constructed by the opening formed on the upper part of the body of apparatus is preferred since it is advantageous in respect of downsizing of the apparatus, quickening of measurement, simplification of measurement, etc.

In the apparatus of the invention, the filter member is constituted by an element that captures a solid component contained in the liquid sample introduced from the liquid sample introduction member. The material constituting the filter member that captures a solid component includes, for example, glass fiber filter paper, cellulose filter paper, nonwoven fabric, nylon cloth, etc. In the present invention, the filter member may be constituted, for example, by a plurality of elements so far as it can capture the solid component contained in the liquid sample. In particular, in the case where the solid component contained in the liquid sample is blood cell, the filter member is preferably made of a material such as glass fiber filter or nylon cloth.

In the apparatus of the invention, the flow passage securing member is provided between the liquid sample introduction member and the filter member to carry a clearance formed so that the permeation of a liquid component in the liquid sample is not prevented by the solid component captured by and accumulated on the filter member. The liquid sample, which contains an object substance, generally tends to pass through in a shortest distance in a region where it can permeate, such as a filter, with the result that the solid component in the liquid sample is apt to be captured by a local region of the filter. In a conventional apparatus, the captured solid component will locally fill the filter or be locally accumulated on the filter according as the liquid sample passes through the filter to locally prevent flow of the liquid sample. On the contrary, in the apparatus of the invention, the flow passage securing member acts as a bypass for the liquid flow so that the solid component can be captured efficiently and at the same time a sufficient amount of permeation of the liquid component toward downstream of the filter member where the analysis member is present can be secured. That is, in the apparatus of the invention, the flow passage securing member secures space around it even if a part of the filter is substantially embedded by the accumulated solid component and has an effect to introduce the liquid flow of liquid sample to the region where the liquid sample in the filter member can pass, i.e., to the region where the accumulation of solid component has not caused hindrance of the flow passage yet.

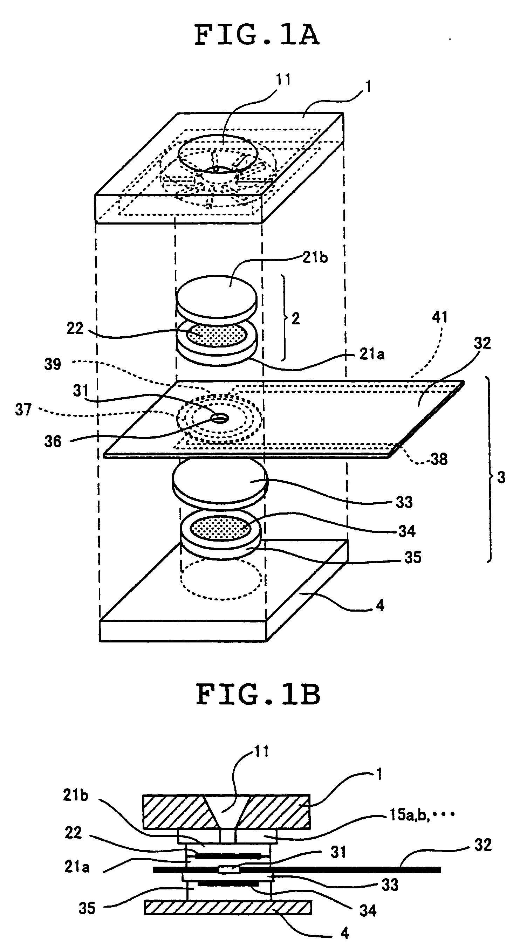

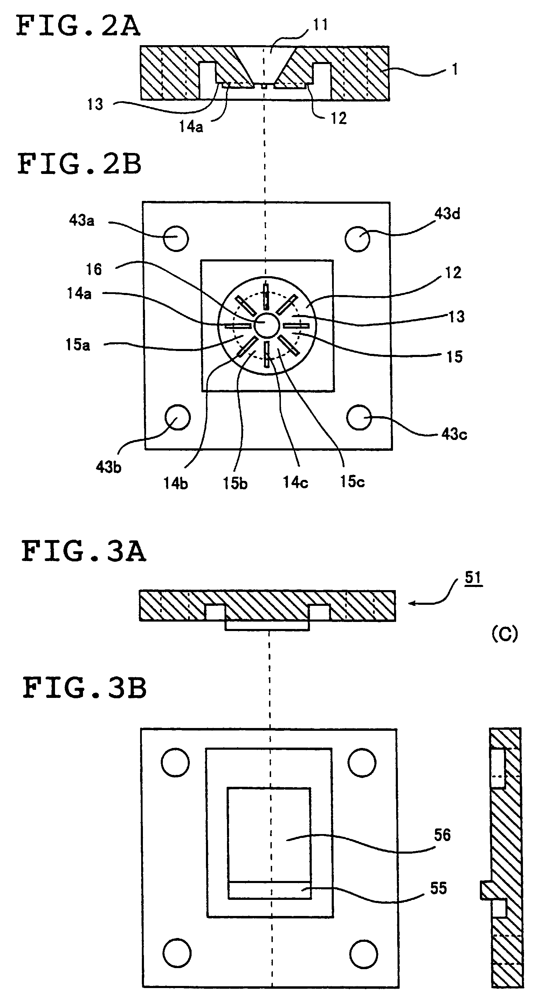

In the apparatus of the invention, the flow passage securing member is constituted, for example, by a clearance formed between a depression formed on a lower surface of the liquid sample introduction member provided on an upper part of the body of apparatus and an upper surface of the filter member. As the flow passage securing member of this form, mention may be made of, for example, the one of which Fig. 1 is a schematic illustration, Fig. 2A is a cross-sectional view, Fig. 2B is a bottom view. As shown in the figures, it is constructed such that it includes a body of apparatus that has an upper cover 1, on a lower part of which is recessed a circular trapezoidal holding member 12 for holding the liquid introduction inlet. On a bottom surface 13 of the holding member 12 are radially projected a plurality of rectangular parallelopiped protrusions 14a, 14b, 14c ..., which have grooves 15a, 15b, 15c, ... in the form of a sector recessed between any adjacent two of them.

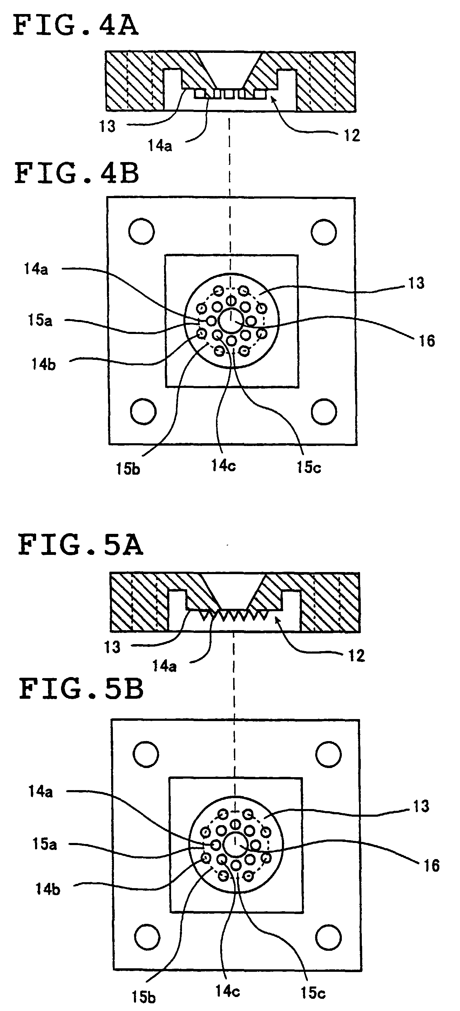

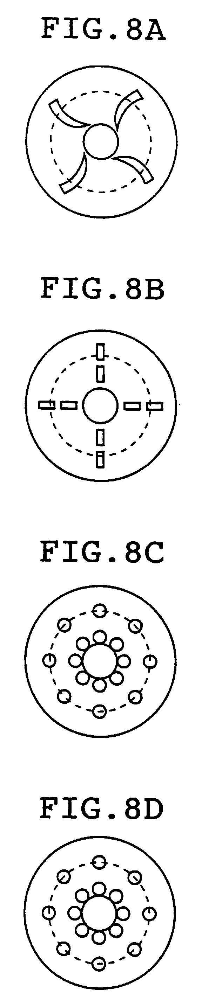

Also, mention may be made of the one of which Figs. 4 to 7 are cross-sectional views and bottom views, respectively, and Figs. 8A to D are only bottom views of the liquid introduction inlet. As shown in the figures, it is constructed such that it includes a circular trapezoidal holding member 12 for the liquid introduction inlet, on a bottom surface 13 of which are projected concentrically or radially centered on a bottom opening 16 of the liquid introduction inlet a plurality of cylindrical or conical protrusions 14a, 14b, 14c, ..., which have grooves 15a, 15b, 15c, ... recessed between any adjacent two of them.

The protrusions or depressions formed in the upper cover can not only construct a flow passage securing member but also play a role of securely holding elements such as a filter member. For example, the MEDIA analysis apparatus referred to hereinbelow is an invaginated type analysis apparatus that comprises an electrode substrate having a communication hole in the center sandwiched by membrane elements or filter elements. Around the communication hole in the center of the electrode substrate are arranged a working electrode as the analysis member and a counter electrode in the ring form. Therefore, in order to guarantee the accuracy of analysis, it is important that the membrane elements or filter paper elements hold the work electrode and counter electrode reliably and at an appropriate intensity. The flow passage securing member formed in the upper cover illustrated in Figs. 1, 2 and 4-7 is preferred in that it can form a flow passage securing member on the upper part of the filter member and it can reliably hold the filter member.

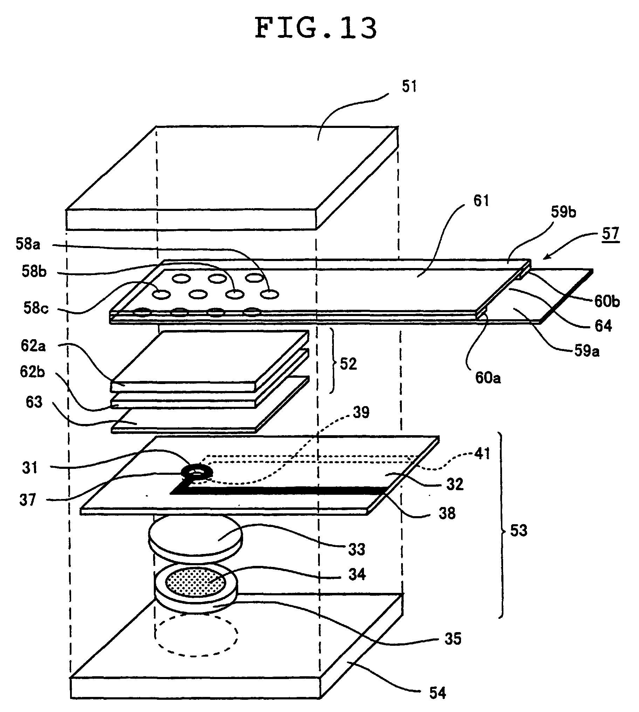

Also, in the apparatus of the invention, as the flow passage securing member may be exemplified the one constructed by an insertion element having a liquid passage, which element is inserted between the upper cover of the body of apparatus and the filter member. As the flow passage securing member of this form, mention may be made of, for example, the one of which Fig. 13 is a schematic illustration, Fig. 3A is a cross-sectional view, Fig. 3B is a bottom view, and Fig. 3C is a side view. As shown in the figures, it is constructed such that it includes a body of apparatus that has an upper cover 51, on a lower part of which is recessed a rectangular parallelopiped insertion element holding member 56 having an insertion element butting member 55, a filter member arranged on a lower part thereof, and an insertion element 57 inserted between the insertion element holding member 56 and the filter member. In the flow passage securing member, the insertion element 57, as shown in Fig. 13, is constituted in such a form that it includes a lower sheet element 59a bored with liquid flow inlets 58a, 58b, 58c, ..., an upper sheet element 59b, and spacers 60a and 60b, these forming a thin layer flow passage 61 through which the liquid sample flows between the lower sheet element 59a and the upper sheet element 59b.

The insertion element having the flow passage securing member includes, for example, porous elements such as a plastic thin plate with a punched-out flow passage securing member, paper subjected to water non-permeating treatment, porous element such as nonwoven fabric, woven fabric or networks made of natural fiber and/or synthetic resin fiber, or filter composed of one of them or two or more of them in combination. For example, as the plastic thin plate with a punched-out flow passage securing member include silicone rubber sheet, polyvinyl chloride sheet, PET film, etc., provided with a punched-out flow passage securing member as examples that can be readily processed. As the porous element, network or filter, there can be exemplified a net made of polyvinyl chloride of 5 to 40 meshes, a nonwoven fabric having a pore size of 10 to 100 µm, etc. The material of the nonwoven fabric described here is not limited. Generally, in the case where captured particles are blood cells, the pore size of the flow passage securing member is preferably 5 µm or more. Also, there can be exemplified as preferred ones a thin layer flow passage or a capillary passage made by a plastic thin plate, paper subjected to water non-permeating treatment, etc. bonded together through a spacer. Such insertion elements not only constitute a flow passage securing member by having appropriate hardness but also can play a role of reliably holding elements such as the filter member.

In the apparatus of the invention, the above construction enables a flow passage securing member to be formed on the surface of the filter. Generally, the liquid sample introduced from the liquid sample introduction member flows into the filter so that it can pass in a shortest distance to cause local clogging with a solid component. However, since the flow passage securing member is present on the surface of the filter, the liquid sample evades the local clogging and readily reaches a different region where no clogging has occurred on the filter.

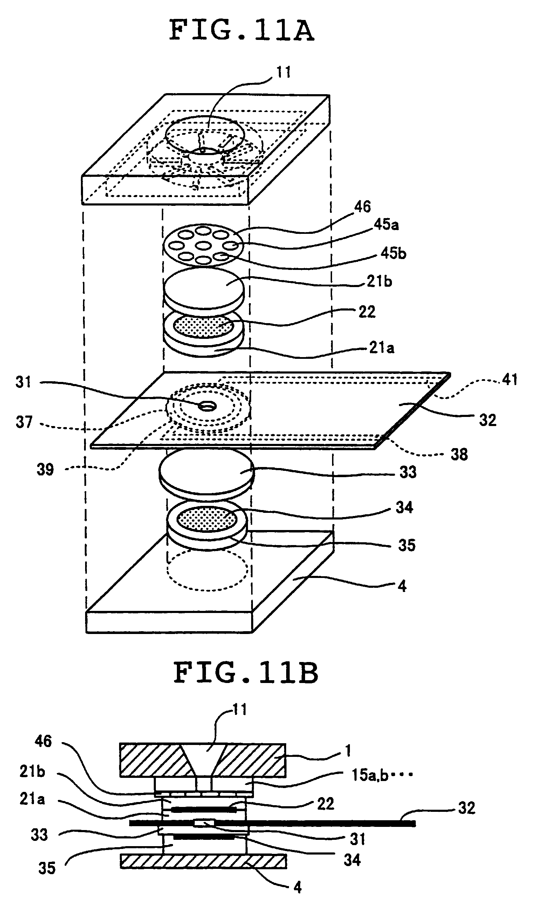

Further, in the apparatus of the invention, as shown in Fig. 11, a liquid non-permeating member 46 bored with at least two flow inlets 45a, etc. may be arranged between the flow passage securing member and the filter member. In the form having a liquid non-permeating element, capturing of the solid component starts on the filter portion of the flow inlet, which is closer to the sample introduction inlet, in the case where there are a plurality of flow inlets or at the flow inlet on the side of the sample introduction inlet in the case where a single flow inlet is present. However, if the liquid flow resistance increases because of its initial region being filled with the solid component, the liquid sample can pass through the flow passage securing member on the liquid non-permeating layer around the flow inlet and readily flow to an other flow inlets or other regions of one flow inlet not filled with the solid component.

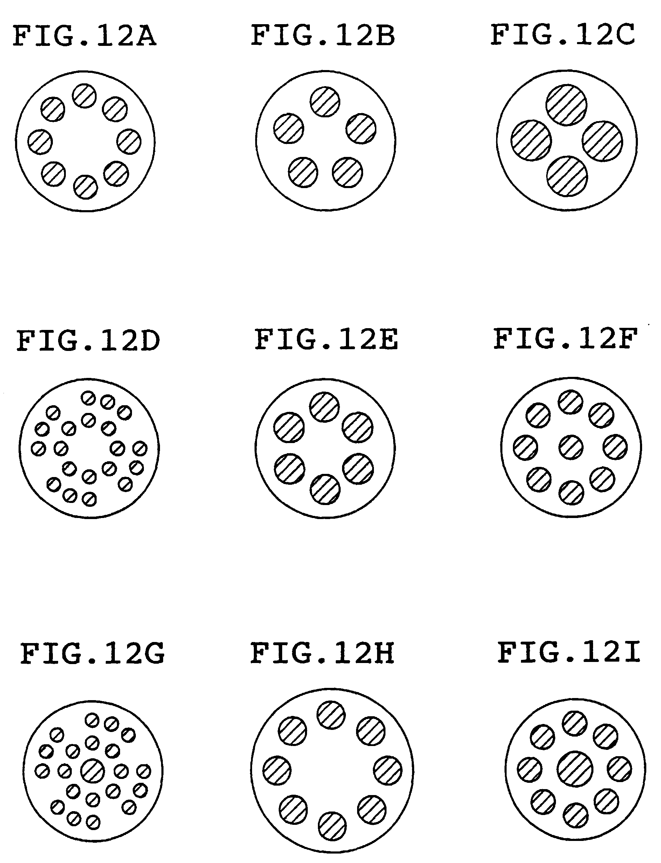

In the liquid non-permeating element, the flow inlet is an opening that allows the liquid sample to flow into the filter member from the flow passage securing member and can be readily formed by punching out the liquid non-permeating element. For example, in an example corresponding to the circular filter member in the MEDIA analysis apparatus referred to hereinbelow, a liquid non-permeating element having a flow inlet as shown in Figs. 12A to I is exemplified as a preferred one.

Also, in the apparatus of the invention, the surface of the filter member may be coated or press-bonded with a liquid non-permeating material to form a liquid non-permeating member having a flow inlet on the upper surface of the filter member.

In the apparatus of the invention, the body of apparatus or the above insertion element can be fabricated from molded products made of various types of materials, such as cutting molded articles, die-molded articles, or sheet laminates made of various materials such as acrylic resin, polyvinyl chloride, glass polystyrene, ABS resin, epoxy resin, paper, etc.

Also, examples of the liquid non-permeating element include those made of various materials such as PET film and polyvinyl chloride thin plate.

In the apparatus of the invention, the analysis member is a part that analyzes the liquid component in the liquid sample. The analysis member measures a signal that is generated or ceased to be generated associated with an object substance and analyzes the amount of the object substance in the liquid sample based on the amount of that signal. The analysis method for the liquid component in the analysis member is selected suitably depending on the kind of object substance, purpose of analysis, properties of the liquid component, etc. For example, there can be exemplified physical analyses such as measurement of conductivity and measurement of refractive index, chemical analyses based on reactions such as complex formation reaction and coloring reaction, enzyme analyses utilizing enzymes such as enzyme sensors, enzyme analyses in which the object substance itself is an enzyme, electrochemical analyses utilizing electron transportation that can be measured with electrodes, fluorophotometrical analyses that can be measured with a fluorometer, emission analyses that can be measured with an emission photometer, color analyses that can be measured by visual judgment or a color difference meter, specific binding analyses utilizing specific binding reaction such as antigen antibody binding reaction or receptor ligand biding reaction, etc.

Also, in the apparatus of the invention, the flow passage securing member provided between the liquid sample introduction member and the filter member avoids the hindrance of the flow of the liquid component caused by the capture of the solid component in the filter member, so that it is sometimes the case that the capture of the solid component in the filter member or suppression of the destruction of the captured solid component is effective. For example, in the case where a whole blood sample is used as an object substance as a liquid sample, in order to effectively avoid the hindrance of flow passage caused by the capture of blood cells, it is sometimes preferable that a blood cell agglutination promoter or a blood cell stabilizer is arranged in the filter member, flow passage securing member or sample introduction member to promote the capture of blood cells in the filter member or suppress the destruction of the captured blood cells. The blood cell agglutination promoter that is used includes, for example, lectins, anti-blood cell antibodies, ionic polymers, etc. The blood cell stabilizer includes, for example, ionic polymers, etc.

To suppress the flow passage hindrance reaction caused by the liquid component itself in the liquid sample, for example, blood coagulation reaction, it is sometimes preferable that a blood coagulation inhibitor be arranged in the filter member, flow passage securing member, liquid sample introduction member or downstream analysis member to suppress blood coagulation in the filter member or the analysis member downstream. As the blood coagulation inhibitor, there can be exemplified heparin, chelating agents such as EDTA, citric acid salts, sodium fluoride, etc.

Further, in the specific binding analysis method represented by the MEDIA method referred to hereinbelow, sometimes enzyme such as peroxidase is used as a label. Also, in enzyme analyses or enzyme sensors, the analysis involves enzyme reaction. In the case where the enzyme in a whole blood sample or enzyme released from blood cells interferes the analysis, it is sometimes preferable that an enzyme inhibitor be arranged in the filter member, flow passage securing member, sample introduction member or analysis member downstream thereof to suppress the enzyme activity that is interfering the analysis in the filter member or analysis member downstream. In the case of the specific biding analysis where peroxidase is used as a labeling enzyme, catalase that consumes hydrogen peroxide, substrate of the enzyme, to generate oxygen is exemplified as an analysis-interfering enzyme. As the catalase inhibitor, there can be exemplified NaN₃ (sodium azide), hydroxylamine, alkylhydroxylamine, cyan compounds, or heavy metal ions such as lead ion, etc.

The apparatus of the invention is suitable for the analysis of liquid samples that possibly contain a solid component. In particular, it is suitable as an analysis apparatus for a whole blood sample in which the solid component is blood cell.

Further, the apparatus of the invention can be applied particularly preferably to the case where specific binding analysis is carried out in a matrix or flow passage composed of a porous membrane as in a specific binding analysis method abbreviated as MEDIA method as disclosed in detail in Published Unexamined Japanese Patent Application No. Hei 5-264552, Published Unexamined Japanese Patent Application No. Hei 9-75748, Published Unexamined Japanese Patent Application No. Hei 9-234097, etc.

The MEDIA method using the apparatus of the invention is a specific binding analysis method that measures an object substance in a liquid sample based on its specific binding reaction, comprising flowing a signal substance generator that participates in a specific binding reaction and generates a signal substance and the liquid sample in a predetermined flow passage in a predetermined direction, generating specific binding reaction of the object substance, forming a distribution of the signal substance generator in the flow passage corresponding to a concentration of the object substance in the liquid sample utilizing the specific binding reaction, generating a signal substance by the signal substance generator distributed in the flow passage, and detecting the generated signal substance by a detection means.

Also, the present invention provides as an apparatus for practicing an MEDIA method a specific binding analysis apparatus comprising a liquid sample introduction member, a flow passage securing member, a filter member, and an analysis member, wherein the analysis member has a flow passage for liquid samples arranged so as to be connected to the filter member and a detection member that detects a distribution of a signal substance generator corresponding to an amount of the object substance in the liquid sample formed in the flow passage by a specific binding reaction of the object substance in the liquid sample with a specific binding substance that specifically binds to the object substance as a signal intensity corresponding to mass transfer by diffusion of the signal substance generated by the signal substance generator.

In the present invention, the specific binding substance is a substance that specifically binds to a specified substance such as the object substance, that is, a substance that can perform specific binding reaction with a specified substance. A substance that has been fragmented such that the specific binding site is not damaged, such as fragments abbreviated as Fab of antibody, Fab, and (Fab)₂, can be used as a specific binding substance. The combinations of the above specified substance with the specific binding substance thereto include an antigen and an antibody thereto, complementary nucleic acid sequences, an effector molecule and a receptor molecule, an enzyme and its inhibitor, an enzyme and a cofactor, an enzyme and a substrate thereof, a compound having a sugar chain and a lectin, or an antibody and an antibody to the antibody, a receptor molecule and an antibody thereto, etc. Also, these combinations are combinations that can be a specific binding substance against the opposite substance mutually.

The specific binding substance also includes those chemically modified to such an extent that the specific binding activity will not be lost or composite substances bonded to other components. As such a specific binding substance, there can be exemplified an antibody or polynucleotide which has been chemically modified with biotin, antibody covalently bonded with avidin, etc. Also, genetically engineered fused proteins composed of an antibody and an enzyme, or an antibody and a receptor, and so on.

Here, a substance that has a moiety functioning as a specific binding substance as a part thereof, such as a signal substance generator referred to hereinbelow may also be called a specific binding substance.

In the present invention, the signal substance generator is a substance that forms a distribution having a correlation with the amount of an object substance in the flow passage and has a part functioning as a specific binding substance and a part that contributes to the generation of a signal substance as will be referred to hereinbelow, i.e., a labeled specific binding substance. The signal substance generator undergoes specific binding reaction, such as binding to a specific binding substance with competing with the object substance or specifically binding to the object substance and at the same time it generates a signal substance either directly or indirectly. That is, the signal substance generator participates in the specific binding reaction between the object substance and a specific binding substance thereto and its distribution in the flow passage varies depending on the amount of the object substance and controls the generation reaction of the signal substance.

The part that functions as a specific binding substance is a part having a structure that serves as a specific binding substance to the object substance or a part having the structure of the object substance or analogs thereof.

The part that contributes the generation of a signal substance is specifically a part constituted by various enzymes used as a label in usual immunoassay, etc.

The signal substance is a substance generated by a reaction in which the signal substance generator participates and that generates a predetermined signal by itself or causes other substance to generate a signal in a detection member referred to hereinbelow.

The substance that participates in the generation of a signal substance could include the above signal substance generator. However, in the present invention, it indicates a substance other than the signal substance generator, mainly a precursor of a signal substance or a substance that contributes to convert the precursor to the signal substance. For example, it includes an electron mediator or a substance that causes to generate an electron mediator, an enzyme substrate, an enzyme cofactor, a hydrogen donor, etc., which will be described hereinbelow.

The substance that participates in the generation of a signal substance means the substances that participate in the generation of signal except for the signal substance in the case where the signal substance does not generate a signal directly and causes other substances to generate a signal or in the case where the signal substance is a substance that generates a signal in the presence of other substances or in cooperation with other substances.

Further, the flow passage means a flow passage in which the liquid sample introduced from the liquid sample introduction member flows or a place where the object substance and the signal substance generator are spread and specific binding reaction depending on the concentration of the object substance takes place.

The liquid sample introduced from the liquid sample introduction member is introduced into the flow passage by external pressure by a pump, etc., external force such as gravitation or spontaneous penetration power.

To enable reproducible introduction of liquid sample into the flow passage in a simple construction of apparatus, it is desirable that the flow passage be constituted by a capillary or a narrow flow passage securing member or the flow passage be constituted by a porous element so that the liquid sample can be introduced into the flow passage by spontaneous penetration power of the liquid sample.

### MODE FOR CARRYING OUT THE INVENTION

Hereafter, the apparatus, specific binding analysis method and specific binding analysis apparatus of the present invention will be explained, based on the construction examples of the apparatus for carrying out specific binding analysis by application of the present invention.

Fig. 1 shows a construction example of the specific binding analysis apparatus of the present invention, basically, having an upper cover 1, a filter member 2, an analysis member 3, and a lower substrate 4.

The upper cover 1 constitutes a liquid sample introduction member, of which Fig. 2A is a cross-sectional view, and Fig. 2B is a bottom view. As shown in the figures, a flow passage securing member is constituted in such a form that it includes, a liquid sample introduction inlet 11 that penetrates an upper surface to a bottom surface of the upper cover 1 to form an introduction passage for a liquid sample, a circular trapezoidal holding member 12 for holding the liquid sample introduction inlet, recessed on a lower part of the cover 1. On a bottom surface 13 of the holding member 12 are radially projected a plurality of rectangular parallelopiped protrusions 14a, 14b, 14c ..., which have grooves 15a, 15b, 15c, ... in the form of a sector recessed between any adjacent two of them.

Also, in the present invention, the flow passage securing member may be constructed in a form of which Figs. 4 to 6 are cross-sectional views and bottom views, respectively, and Figs. 8A to D are only bottom views of the liquid introduction inlet. As shown in the figures, it is constructed such that it includes a circular trapezoidal holding member 12 for the liquid introduction inlet, on a bottom surface 13 of which are projected concentrically or radially centered on a bottom opening 16 of the liquid introduction inlet, a plurality of cylindrical or conical protrusions 14a, 14b, 14c ..., which have grooves 15a, 15b, 15c, ... recessed between any adjacent two of them.

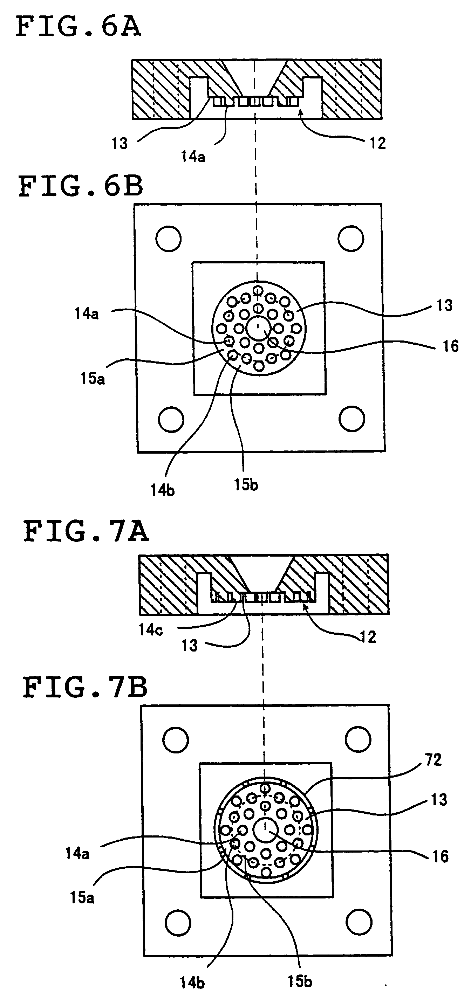

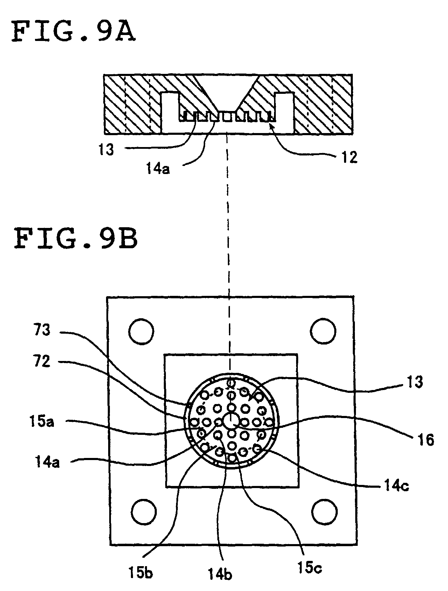

Further, the flow passage securing member may be constructed in a form of which Figs. 7 and 9A are cross-sectional views and Fig. 9B is a bottom view. As shown in the figures, it is constructed such that it includes a conical-shaped holding member 12 for the liquid introduction inlet, on a bottom surface 13 of which are projected in the form of three concentric circles centered on a bottom opening part 16 of the liquid introduction inlet, a plurality of cylindrical protrusions 14a, 14b, 14c ..., which have grooves 15a, 15b, 15c ... recessed between any adjacent two of them, and further, the protrusions 14a, 14b, 14c ..., are surrounded by a plurality of arcuate frames 72 formed along a periphery of the bottom surface 13 through a plurality of grooves 73.

The filter member 2 is constructed by a glass fiber filter medium 21 impregnated with an enzyme-labeled specific binding substance and an electron mediator, a glass fiber filter paper 21b provided with a liquid non-permeating seal member 22 on its upper surface, on which surface a glass fiber filter paper 21a is mounted, and impregnated with NaN₃.

The analysis member 3 is constructed by, an electrode substrate 32 having a communication hole 31 perforated from its upper surface to its lower surface, an antibody insolubilized porous membrane 33 arranged under the electrode substrate, and a water absorption member glass fiber filter paper 35 having on its upper surface a dialysis membrane seal member 34 and impregnated with an enzyme substrate. In a lower surface of the electrode substrate 32 are formed an annular working electrode (detection member) 37 concentrically surrounding a lower opening of a communication hole 36, and a working electrode terminal 38 connected to the working electrode 37, and further, on a lower surface of the electrode substrate are formed an annular counter electrode 39 concentrically surrounding the working electrode 37, and a counter electrode terminal 41 connected to the counter electrode 39.

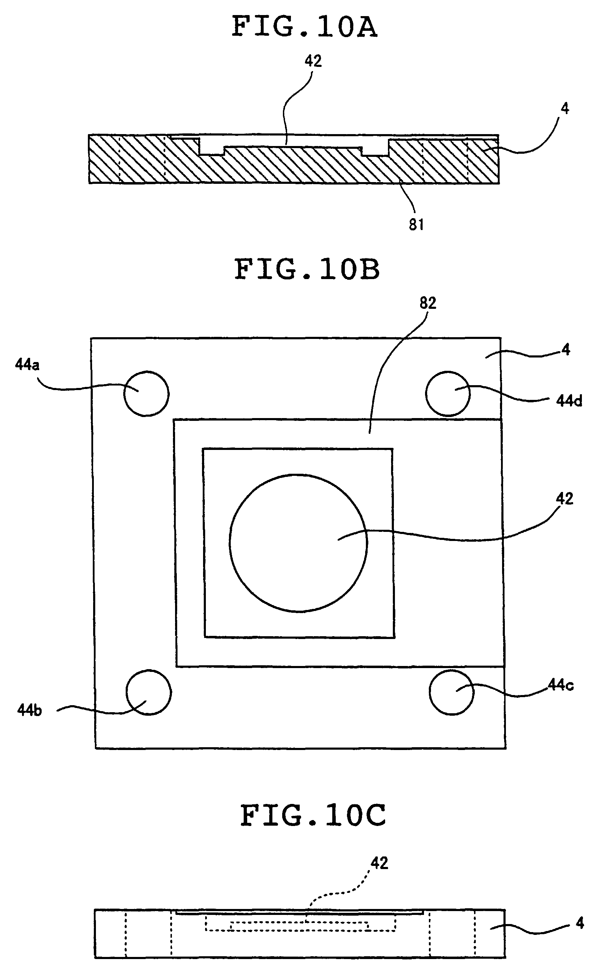

Further, the lower substrate 4, of which Fig. 10A is a front view, Fig. 10B is a bottom view, and Fig. 10C is a side view, has a cylindrical seat 42 on which the water absorption member glass fiber filter paper 35 of the above analysis member is mounted, projected in a depression 82 recessed in a base 81 for fixing the electrode. At the four corners of the base 81 are perforated assembly holes 44a, 44b, 44c, and 44d corresponding to assembly holes 43a, 43b, 43c, and 43d perforated at four corners of the upper cover 1. The lower substrate 4 is not limited to the exemplified forms, but may be of any form so far as it can be regulated with respect to the arrangement of elements to be laminated and distances between the laminated layers.

The apparatus shown in Fig. 1, as shown in Fig. 1B, can be assembled by laminating on the lower substrate 4 in order of the water absorption member glass fiber filter paper 35 impregnated with an enzyme substrate and having a dialysis membrane seal member 34 on its upper surface, the specific binding substance-insolubilized porous membrane 33, the electrode substrate 32, the glass fiber filter paper 21b, the glass fiber filter paper 21a, and the upper cover 1, and inserting bolts (not shown) into the assembly holes 43a, 43b, 43c, and 43d perforated at the four corners of the upper cover 1, and the assembly holes 44a, 44b, 44c, and 44d perforated correspondingly at the four corners of the lower substrate 4, and fixing with nuts (not shown).

In the apparatus shown in Fig. 1, for example, a whole blood sample, is introduced, as a liquid sample, from the liquid sample inlet 11 of the upper cover (Fig. 2) having a role of the liquid sample introduction member. The introduced liquid sample passes the flow passage securing member, to capture a solid component, for example, erythrocytes, etc. in the filter member, and only a liquid component is subjected to analysis by specific binding reaction of the object substance in the analysis member. In this case, the flow passage securing member constructed by depressions 15a, 15b, 15c ... recessed between any adjacent two of a plurality of protrusions 14a, 14b, 14c ... functions so that the permeation of the liquid component in the liquid sample is not inhibited by the solid component captured by and accumulated on the filter member. Generally, the liquid sample which is an object substance, tends to pass in a permeable region such as a filter, generally by flowing in a shortest distance, with the result that the solid component is apt to be captured by a local region of the filter. In the conventional apparatus, the captured solid component will locally fill the filter, or be locally accumulated on the filter according as the liquid sample passes through the filter, to prevent local flow of the liquid sample. On the contrary, in the present apparatus, the flow passage securing member acts as a bypass for the liquid flow, so that the solid component can be captured efficiently, and at the same time a sufficient amount of permeation of the liquid component toward downstream of the filter member where the analysis member is present can be secured. That is, in the apparatus of the invention, the flow passage securing member secures space around it even if a part of the filter is substantially embedded by the accumulated solid component, and has an effect to introduce the liquid flow of liquid sample to the region where the liquid sample can pass, i.e., to the region where the accumulation of solid component has not caused hindrance of the flow passage yet.

The apparatus shown in Fig. 11, has a construction similar to that shown in Fig. 1. It has a construction such that it further comprises a liquid non-permeating element 46 having a plurality of flow inlets 45a, 45b, 45c ..., that cause the liquid sample to flow into the filter member from the flow passage securing member, between a plurality of protrusions 14a, 14b, 14c ..., formed on the bottom surface 13 of the holding member 12 for the liquid introduction inlet of the upper cover 1 and the filter member 2.

The liquid non-permeating element 46 is not limited to the one having the construction shown in Fig. 11, but may be of the form shown in Figs. 12A to I, for example.

In the apparatus shown in Fig. 11, for example, a whole blood sample is introduced as a liquid sample, from the liquid sample inlet 11 of the upper cover having a role of the liquid sample introduction member; The introduced liquid sample passes the flow passage securing member to capture a solid component, for example, erythrocytes, etc. in the filter member, and only a liquid component is subjected to analysis by specific binding reaction of the object substance in the analysis member. Then, a plurality of depressions 15a, 15b, 15c ..., recessed between any adjacent two of a plurality of protrusions 14a, 14b, 14c ..., and the above liquid non-permeating element 46 act as a flow passage securing member as shown in the above Fig. 1. In the case where the liquid non-permeating element 46 has a plurality of flow inlets, the capture of the solid component starts at the filter part of the flow inlet, which is closer to the sample introduction inlet. Alternatively, in the case where only one flow inlet is present, it starts at the flow inlet on the side of the sample introduction inlet. However, this is advantageous in that, if the liquid flow resistance increases because of its initial capturing region being filled with the solid components, the liquid sample can pass through the flow passage securing member on the liquid non-permeating layer around the flow inlet, and readily flow to an other flow inlets or other regions of one flow inlet not filled with the solid component.

Further, the apparatus shown in Fig. 13, which is another example of the specific binding analysis apparatus of the present invention, basically includes an upper cover 51, a filter member 52, an analysis member 53, and a lower substrate 54. Basically, the filter member 52, the analysis member 53, and the lower substrate 54 are similar in construction to those in the apparatus shown in the above Fig. 1, and the part or site designated by the same reference numeral as those shown in Fig. 1 indicate the same part or site shown in Fig. 1.

In the apparatus shown in Fig. 13, the upper cover 51, unlike the apparatus shown in Fig. 1, is a flat plate element having no liquid sample introduction inlet, of which Fig. 3A is its cross-sectional view, Fig. 3B is its bottom view, and Fig. 3C is its side view. As shown in the figures, it has recessed in its lower part a rectangular parallelepiped insertion element holding member 56 having an insertion element butting member 55 on one end thereof. The flow passage securing member in this apparatus is constructed by the rectangular parallelepiped insertion element holding member 56 recessed under the upper cover 51, having the insertion element butting member 55 on one end thereof, and an insertion element 57 inserted between the insertion element holding member 56 and the filter member provided thereunder. The insertion element 57, as shown in Fig. 13, is constructed in such a form that a thin layer flow passage 61 in which the liquid sample flows is formed by a lower sheet element 59a perforated with liquid flow inlets 58a, 58b, 58c, ..., an upper sheet element 59b, spacers 60a, 60b provided in the longitudinal direction of both the elements between the lower sheet element 59a and the upper sheet element 59b.

Also, in this apparatus, the filter member 52 is constructed by two laminated nonwoven fabrics 62a and 62b and a filter medium 63, for example, blood cell filter paper, provided under the nonwoven fabrics 62a and 62b.

Also, the analysis member 53 includes an electrode substrate 32 having a communication hole 31 penetrating from its upper surface to its lower surface, an antibody-insolubilized porous membrane 33 provided under the electrode substrate, and water absorption member cellulose filter paper 35 impregnated with an enzyme substrate and having a dialysis membrane seal member 34 on its upper surface. On the lower surface of the electrode substrate 32 are formed an annular working electrode (detection member) 39 concentrically surrounding an upper opening of the communication hole 31 and a working electrode terminal 41 connected to the working electrode 39. On the upper surface of the electrode substrate are formed an annular counter electrode 37 concentrically surrounding a lower opening of the communication hole 31 and a counter electrode terminal 38 connected to the counter electrode 37.

Further, the lower substrate 54 has a projected, cylindrical seat 42 on which the water absorption member cellulose filter paper 35 of the above analysis member is mounted (Fig. 10).

The apparatus shown in Fig. 13 can be assembled by laminating on the lower substrate 54 in order the water absorption member cellulose filter paper 35 impregnated with an enzyme substrate and having a dialysis membrane seal member 34 on its upper surface, the antibody-insolubilized porous membrane 33, the electrode substrate 32, the filter medium 63, the nonwoven fabrics 62a and 62b, and the insertion element 57 and the upper cover 51 in order and fixing these using bolts and nuts in the assembly holes (not shown) perforated at the four corners of the upper cover 51 and the assembly holes (not shown) perforated correspondingly at the four corners of the lower substrate 54.

In the apparatus shown in Fig. 13, a whole blood sample, for example, is introduced as a liquid sample into a thin layer flow passage 61 from a terminal opening 64 of the insertion element 57 having a role of the flow passage securing member. The introduced liquid sample passes through the thin layer flow passage 61 and the liquid flow inlets 58a, 58b, 58c, ... to capture a solid component, for example, erythrocytes, etc. in the filter member and only a liquid component is subjected to analysis by specific binding reaction of the object substance in the analysis member. Then, the flow passage securing member constructed by the insertion element 57 perforated with the liquid flow inlets 58a, 58b, 58c, ... functions so that the permeation of the liquid component in the liquid sample is not inhibited by the solid component captured by and accumulated on the filter member. That is, according as the liquid sample passes through the filter, the captured solid component will locally fill the filter or be locally accumulated on the filter. Then, the thin layer flow passage 61 and the liquid flow inlets 5r8a, 58b, 58c, ... act as a bypass of the liquid flow to enable efficient capture of the solid component and at the same time secure a sufficient amount of permeation of the liquid component toward downstream of the filter member where the analysis member is present.

A preferred example of the insertion element playing the role of the flow passage securing member includes a clogging preventing filter. For example, mention may be made of the one having the following construction.
a) The construction in which a reagent component (labeled substance, enzyme substrate, electron mediator, buffer component, surfactant, etc.) is impregnated and dried in a blood cell capturing filter made of a glass fiber filter paper to arrange the reagent component in the filter and a clogging preventing filter as a flow passage securing member is arranged thereon.
b) The construction in which a portion of a reagent component (a labeled substance, an enzyme substrate, an electron mediator, a buffer component, a surfactant, an enzyme inhibitor, a blood coagulation inhibitor, a blood coagulation promoter, etc.) is arranged in a dry state upstream of the blood cell capturing filter (glass fiber filter paper). In this construction, the reagent component is arranged in a dry state without any carrier at the sample introduction inlet upstream of the clogging preventing filter. In particular, those reagent components that are desired to be added prior to capturing blood cells (surfactants, enzyme inhibitors, blood coagulation inhibitors, blood agglutination promoters, etc.) are preferably arranged in this form.
c) The construction in which a clogging preventing filter is arranged above the blood cell capturing filter (cellulose filter paper), a portion of the reagent component is arranged in a dry state upstream of the blood cell capturing filter, and another portion of the reagent component is arranged in a dry state on the water absorption member filter paper. In particular, it is preferred that reagent components that are desired not to contact with the captured blood cells (enzyme substrates) be arranged in this manner on the water absorption member filter paper.
d) The construction in which locations of the sample introduction inlet and the communication member to the analysis member are dislocated in the vertical direction. In this construction, dislocation of the sample introduction inlet and communication member (communication holes or communication terminals) can effectively avoid the hindrance of the flow passage by the captured particles.

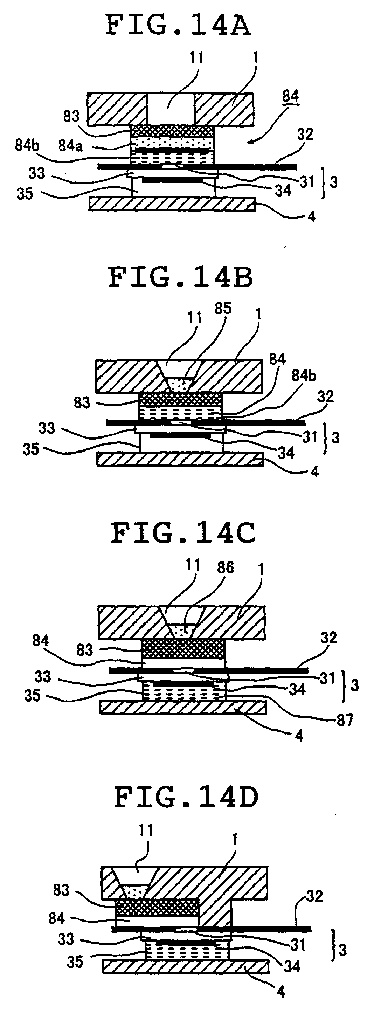

Specific examples of the constructions a) to d) above are shown in Figs. 14A to D. Figs. 14A to D show preferred examples of the analysis apparatus having the flow passage securing member (clogging preventing filter) and capturing filter in the present invention. The figures show positional relationship between the flow passage securing member using the clogging preventing filter and the capturing filter and the arrangement of a reagent component incorporated in the analysis apparatus in a dry state as a cross-sectional view of a specific binding analysis apparatus similar to the apparatus shown in Fig. 1B or Fig. 11B. The elements similar to those in the apparatus shown in Fig. 1B or Fig. 11B are designated by the same reference numerals as in Fig. 1B or Fig. 11B.

Figs. 14A to D are schematic cross-sectional views showing specific examples of the analysis apparatus having the constructions a) to d) above, respectively. As in the apparatus shown in Fig. 1B or Fig. 11B, the apparatus includes an upper cover 1, an analysis member 3, and a lower substrate 4. The analysis member 3 is constructed by an electrode substrate 32 having a communication hole 31 penetrating the substrate from its upper surface to its lower surface, an antibody-insolubilized porous membrane 33 arranged under the electrode substrate, and a water absorption member cellulose filter paper 35 having on its upper surface a dialysis membrane seal member 34. In a lower surface of the electrode substrate 32 are formed an annular working electrode (detection member) concentrically surrounding a lower opening of a communication hole 36 and a working electrode terminal connected to the working electrode. Further, on a lower surface of the electrode substrate are formed an annular counter electrode concentrically surrounding the working electrode and a counter electrode terminal connected to the counter electrode.

The apparatus shown in Fig. 14A has a construction such that between the upper cover 1 and the electrode substrate 32 in order from the side of the upper cover 1, a clogging preventing filter 83 as a flow passage securing member, and a blood cell capturing filter 84 made of a glass fiber filter paper under the clogging preventing filter 83 are laminated. The blood cell capturing filter 84 has the construction in which sandwiching the liquid non-permeating seal member 22 are provided a labeled substance-impregnated member 84a impregnated with a labeled substance as a main reagent component above the seal member and an enzyme substrate-impregnated member 84b impregnated with an enzyme substrate as a main reagent component under the seal member.

The apparatus shown in Fig. 14B has a construction such that between the upper cover 1 and the electrode substrate 32 in order from the side of the upper cover 1, the clogging preventing filter 83 as a flow passage securing member, and the blood cell capturing filter 84 made of a glass fiber filter paper under the clogging preventing filter 83 are laminated. Further, at the sample introduction inlet 11 of the upper cover 1 is provided a labeled substance 85 dried without any carrier. The blood cell capturing fitter 84 is impregnated with an enzyme substrate as a main reagent component to form an enzyme substrate-impregnated member 84b. This apparatus is preferable particularly in the case where those reagent components that are desired to be added prior to capturing blood cells (surfactants, enzyme inhibitors, blood coagulation inhibitors, blood agglutination promoters, etc.) are arranged.

The apparatus shown in Fig. 14C has a construction such that it includes laminated between the upper cover 1 and the electrode substrate 32 in order from the side of the upper cover 1, the clogging preventing filter 83 as a flow passage securing member, and the blood cell capturing filter 84 made of a cellulose filter paper under the clogging preventing filter 83. At the sample introduction inlet 11 of the upper cover 1 is provided non-carrier dried product 86 containing a labeled substance and blood cell agglutinin. The water absorption member filter paper 35 is impregnated with an enzyme substrate to form an enzyme substrate-impregnated member 87. This apparatus is preferable particularly in the case where those reagent components that are desired not to contact with the captured blood cells (enzyme substrates, etc.) are arranged.

The apparatus shown in Fig. 14D has a construction such that it includes laminated between the upper cover 1 and the electrode substrate 32 in order from the side of the upper cover 1, the clogging preventing filter 83 as a flow passage securing member, and the blood cell capturing filter 84 made of a glass fiber filter paper under the clogging preventing filter 83. Similarly to the apparatus shown in Fig. 14C, at the sample introduction inlet 11 of the upper cover 1 is provided non-carrier dried product 86 containing a labeled substance and blood cell agglutinin. The water absorption member filter paper 35 is impregnated with an enzyme substrate to form an enzyme substrate-impregnated member 87. At the same time, the relative positions in the horizontal direction of a lower opening of the sample introduction inlet 11 and the communication hole 31 of the analysis member are dislocated. In this apparatus, the dislocation of the sample introduction inlet 11 and communication member (communication holes or communication terminals) can effectively avoid the hindrance of the flow passage by the captured particles.

The apparatuses shown in Figs. 14A to D are preferred examples and the positional relationship between the clogging preventing filter as a flow passage securing member and the capturing filter and the arrangement of reagent component, etc. are not limited to these examples.

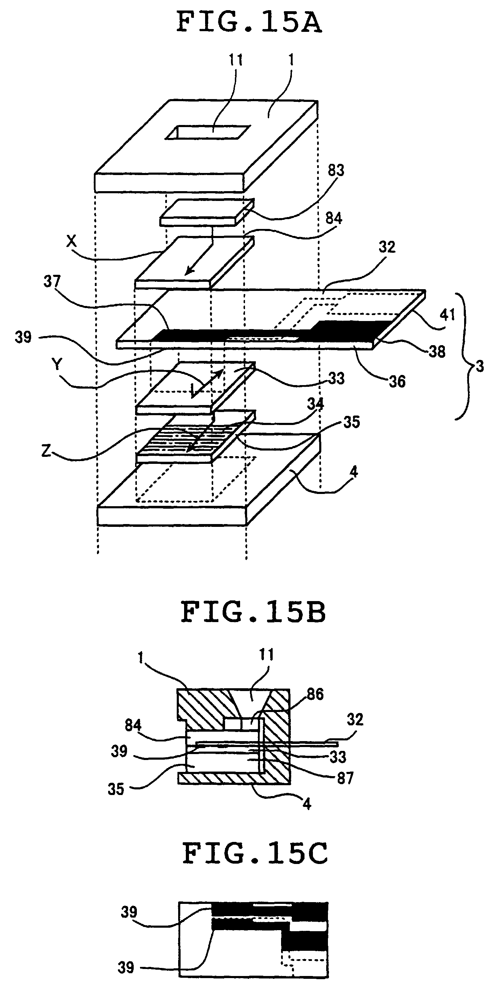

Figs. 15A to C show a construction example of the specific binding analysis apparatus corresponding to the construction shown in Fig. 14D in which the locations of the sample introduction inlet 11 and the communication member to the analysis member 3 are dislocated.

Fig. 15A is a diagram showing the construction of each element, Fig. 15B shows an assembly construction example, and Fig. 15C is an enlarged diagram showing the rear surface of the electrode substrate.

In the apparatus shown in Fig. 15, the analysis member 3 is constructed by an electrode substrate 32 having a communication hole 31 penetrating it from its upper surface to its lower surface, an antibody-insolubilized porous membrane 33 provided under the electrode substrate, and a water absorption member cellulose filter paper 35 impregnated with an enzyme substrate. On the lower surface of the electrode substrate 32 are formed a working electrode (detection member) 39 contacting the communication hole or communication terminal 36 and a working electrode terminal 41 connected to the work electrode 39 while on the upper surface of the electrode substrate are formed a counter electrode 37 contacting the communication hole or communication terminal and a counter electrode terminal 38 connected to the counter electrode 37.

As described above, the dislocation of the sample introduction inlet and the communication member (communication hole or communication terminal) can avoid the hindrance of the flow passage by the captured particles just under the sample introduction inlet. Further, the dislocation of the sample introduction inlet and communication member increases surface area of the captured filter and enables the flow passages indicated by arrows X, Y and Z to be constructed longer, resulting in an increase in both the efficiency of capture and the efficiency of avoiding the hindrance of the flow passage as well as an increase in the efficiency of admixture of a sample and a reagent in the flow passage. Further, in the case of the specific binding analysis apparatus in which the electrode as a detection member is arranged around the communication member, the sample introduction inlet, an open space, is remote form the communication member, which enables the detection member in the vicinity of the communication member to be held more firmly.

Hereinafter, the present invention will be described more concretely by examples. However, the present invention should not be limited to the examples.

### (Embodiment 1)

### 〈Preparation of elements〉

Hereinafter, the dimensions of a quadrate element (A×B) were described such that A is in the direction perpendicular to the liquid flow and b is in the direction parallel to the liquid flow.

### 〈Preparation of horseradish peroxidase (HRPO)-labeled anti-E2 antibody〉

Horseradish peroxidase (HRPO)-labeled anti-E2 antibody was prepared according to the method of Jane J. Zara et al. described in Analytical Biochemistry 194, 156-162 (1991) using horseradish peroxidase (HRPO) (Toyoboseki).

### 〈Preparation of E2-6CMO-γ-globulin〉

E2-6CMO (Sigma) activated with N-hydroxysuccinimide (Wako Pure Chemical Industries, Ltd.) and 1-ethyl-3-(3-dimethylaminopropyl)carbodiimide hydrochloride (Wako Pure Chemical Industries, Ltd.) was added to a solution of γ-globulin (Sigma), and the mixture was stirred at 4°C for one night. Then, E2-6CMO-γ-globuiin was purified using Sephadex-G25 (Pharmacia Biotech, Inc.).

### 〈Production of antigen-insolubilized membrane〉

2.0 mg/ml of E2-6CMO-γ-globuiin antigen solution was prepared using phosphate buffer saline (76 mM PB - 76 mM NaCl, pH 6.4, hereafter abbreviated as "PBS"). A 10 mmφ mixed cellulose porous membrane (Millipore, Inc.) was dipped in an antigen solution and the mixture was shaken at 25°C for one night to insolubilize the antigen. After washing the antigen insolubilized membrane, it was dipped in a 1 % casein (Wako Pure Chemical Industries, Ltd.)/PBS solution to carry out blocking. Then, the membrane was washed and dried under reduced pressure to obtain an antigen insolubilized membrane.

### 〈Production of HRPO-labeled E2 antibody-electron mediator THEPD lyophilizate〉

As an electron mediator, N,N,N',N'-tetrakis(2-hydroxyethyl) -*p*-phenylenediamine (Mochida Pharmaceutical Co., Ltd., abbreviation: THEPD) was used.

Using a 12mM THEPD - 5mg/ml anti-erythrocyte antibody (Organon Teknika Co.) - 5% normal rabbit serum (Nippon Seibutsu Zairyo, abbreviation: NRS) - 5% maltose (Wako Pure Chemical Industries, Ltd.) - 160 U/ml heparin (Wako Pure Chemical Industries, Ltd.) - 100 mM sodium chloride - 40 mM phosphate buffer (abbreviation: PB) (pH: 6.0) solution, a diluted solution of HRPO-labeled anti-E2 antibody was prepared. 30 µl each of the HRPO-labeled anti-E2 antibody diluted solution was dripped on a tray and lyophilized.

### 〈Production of catalase inhibitor (NaN₃) lyophilizate〉

10 mM NaN₃ (Wako Pure Chemical Industries, Ltd.) - 160 U/ml heparin - 10 mM PB (pH: 6.0) was prepared. This solution was applied to nonwoven fabric cut to 10×14 mm in 70 µl. Then, this was frozen and lyophilized on a tray to obtain a catalase inhibitor (NaN₃) lyophilizate.

### 〈Production of enzyme substrate (hydrogen peroxide) lyophilizate〉

A solution of 300 mM hydrogen peroxide (Wako Pure Chemical Industries, Ltd.) - 300 mM hydantoic acid (Tokyo Kasei) (pH: 6.0) - 20mM potassium guaiacolsulfonate (Tokyo Kasei) - 160 U/ml heparin was prepared and was applied to 8 mmφ cellulose filter papers (Whatman, 3MMChr), arranged on a tray, in 15 µl/sheet. They were frozen and lyophilized on a tray to form hydrogen peroxide lyophilizates.

### 〈Production of a flow passage securing member element having an HPRO-labeled antibody - THEPD lyophilizate〉

A PET film (thickness: 0.1 mm) was cut to 12 mm × 25 mm and a 2 mm wide adhesive tape was bonded to its both ends which are parallel to the liquid flow, to fix a 2 mm wide polyvinyl chloride film (thickness: 0.3 mm) as an insertion body for forming a flow passage securing member.

Further, two adhesive tapes were bonded in the direction perpendicular to the polyvinyl chloride film to form a depression for placing lyophilizates between the two adhesive tapes.

The above-described flow passage securing member element having a depression for placing lyophilizates was placed on a cooled tray and a solution of HRPO-labeled anti-E2 antibody - 12mMTHEPD - 5mg/ml anti-erythrocyte antibody - 5% NRS - 5% maltose - 160 U/ml heparin - 100 mM NACl - 40 mM PB (pH: 6.0) was dispensed in 30 µl on to the depression for placing lyophilizates, frozen and lyophilized, to obtain HRPO-labeled antibody - THEPD lyophilizates.

### 〈Production of an analysis apparatus having a flow passage securing member having incorporated therein HRPO-labeled antibody - THEPD lyophilizates〉

The adhesive tapes that formed the depression in the flow passage securing member component having HRPO-labeled antibody - THEPD lyophilizates were peeled off.

Then, 2 mm wide adhesive double coated tape was pasted in the direction parallel to the liquid flow.

On the other hand, a PET film (thickness: 0.1 mm) cut to 12 mm × 35 mm was perforated with 12 (3×4) holes of 2 mmφ as flow inlets.

Then, the PET film with these flow inlets and the above-described flow passage securing member element having the HRPO-labeled antibody - THEPD lyophilizates were bonded together, to form a thin layer flow passage having a flow passage securing member in which the lyophilizates were arranged inside.

### 〈Production of a whole blood dry system analysis apparatus〉

As shown in Fig. 13, a cellulose filter paper 35 (8 mmφ) serving as an enzyme substrate impregnating member formed by impregnating hydrogen peroxide was fixed to a lower substrate 54 made of acrylic resin, and a 5 mmφ circular liquid non-permeating seal 34 as a dialysis membrane seal member was bonded in the center part of the upper surface of the enzyme substrate impregnating member. On this was mounted an antigen insolubilized membrane 33 with its center being aligned. Further, an electrode substrate (electrode A) 32 was superimposed so that the center of the communication hole could be aligned. In a communication hole 31 of the electrode was inserted a cellulose filter paper 5C (ADVANTEC) cut to 3 mmφ. Then, an adhesive double coated tape was bonded to fix the cellulose filter paper 5C (10×14 mm, ADVANTEC) 63 on the electrode plate. Also, on the cellulose filter paper 5C were laminated two layers 62a and 62b of NaN₃ lyophilized nonwoven fabric (10×14 mm).

### Particulars of Electrode A

Carbon working electrode:
   3 to 5 mmφ ring-like electrode (lower surface)
Silver counter electrode:
   3.5 to 7 mmφ ring-like electrode (upper surface)
Communication hole: 3 mmφ

In this apparatus, an adhesive double coated tape was bonded to the lower surface of the upper cover 51 made of acrylic resin to fix the previously fabricated thin layer capillary flow passage (cf. Fig. 3). Further, three spacers (thickness: 0.5 mm) were inserted in each screw position in the upper cover so that they were parallel to the liquid flow. The upper cover 51 was put on the lower substrate 54 made of acrylic resin having laminated thereon the above cellulose fiber filter paper 35, antigen insolubilized membrane 33, electrode substrate (electrode A) 32, cellulose filter paper 5C (10×14 mm) 63, and NaN₃ lyophilized nonwoven fabrics (10×14 mm) 62a and 62b, such that the 3×4 holes of flow inlets in the thin layer capillary flow passage were directed downward (toward the surface of the nonwoven fabric), and the four corners of the upper cover and of the lower substrate were screwed to assemble an analysis apparatus as shown in Fig. 13.

Also, an analysis apparatus was assembled in the same manner as above except that the HRPO-labeled antibody - THEPD lyophilizate was not incorporated in the insertion element.

### 〈Analysis of whole blood samples〉

In the analysis apparatus in which HRPO-labeled antibody - THEPD lyophilizate was incorporated in the insertion element, 140 µl of a sample obtained by adding each concentration of E2 to whole blood exsanguinated using a heparin-coated blood collecting tube was injected into the thin layer capillary flow passage and the electric current was measured.

In the analysis apparatus in which no HRPO-labeled antibody - THEPD lyophilizate was incorporated in the insertion element, the HRPO-labeled antibody - THEPD lyophilizate was dissolved in whole blood to which each concentration of E2 was added, and then 140 µl of the sample was injected in the thin layer before the electric current was measured. Just before the addition of the sample liquid, a voltage of -150 mV with respect to the counter electrode was applied to the working electrode. Measurement of the electric current was performed using an electric current measuring circuit (Mochida Pharmaceutical Co., Ltd.) and electric current value was measured at intervals of 1 second.

### (Results)

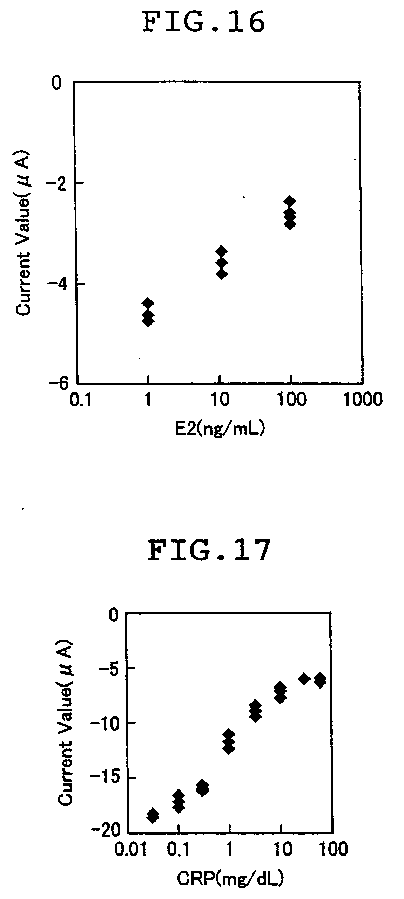

As a result, in the analysis apparatus in which HRPO-labeled antibody - THEPD lyophilizate was incorporated in the insertion element, and the analysis apparatus in which no HRPO-labeled antibody - THEPD lyophilizate was incorporated in the insertion element, good electric current responses to E2 concentration were obtained without causing clogging by blood cells as shown in Fig. 16.

### (Embodiment 2)

### 〈Preparation of Tween 20-treated GA100 glass fiber filter paper〉

Glass fiber filter paper GA100 (ADVANTEC) was dipped in an aqueous 0.2% Tween 20 solution at room temperature for one night. Further, it was washed with distilled water and heat-dried at 80°C.

### 〈Production of HRPO-labeled CR2〉

HRPO to which a maleimide group was introduced with Sulfo-SMCC (PIERCE) was connected to CRP, to which a sulfohydryl group was introduced with Traut's reagent (PIERCE), and the product was purified through a Superdex 200 column (Pharmacia Biotech) to obtain HRPO-labeled CRP.

### 〈Production of HRPO-labeled CRP - THEPD glass fiber filter paper〉

An HRPO-labeled CRP diluted solution was prepared using a solution of 2 mM THEPD - 5% NRS - 10% lactose - 160 U/ml heparin - 100 mM NaCl -50 mM piperazine - 1,4-bis(2-ethanesulfonic acid (hereinafter abbreviated as "PIPES", Wako Pure Chemical Industries, Ltd.) (pH: 7.4). 120 µl of this diluted solution was applied to the Tween 20-treated GA100 glass fiber filter paper cut to a 11 mmφ circle and lyophilized to obtain an HRPO-labeled CRP - THEPD glass fiber filter paper.

### 〈Production of mouse monoclonal anti-CRP antibody-insolubilized membrane〉

0.2 mg/ml of mouse monoclonal anti-CRP antibody solution was prepared using phosphate buffer saline (76 mM PB - 76 mM NaCl, pH 6.4, hereinafter, abbreviated as "PBS"). A 10 mmφ disk-shaped porous membrane was dipped in the antibody solution to insolubilize the antibody. Then, after washing the membrane, it was dipped in a 1% casein/PBS solution at 25°C for 30 minutes to effect blocking. Further, after washing the membrane, it was dried under reduced pressure to obtain mouse monoclonal anti-CRP antibody-insolubilized membrane.

### 〈Production of hydrogen peroxide lyophilizate〉

A solution of 50 mM hydrogen peroxide - 100 mM hydantoic acid (pH 6.0) - 20 mM potassium guaiacolsulufonate - 160 U/m heparin was prepared, and applied to 10 mmφ glass fiber filter paper GB100R (ADVANTEC) in an amount of 75 µl per sheet, followed by lyophilization to obtain a hydrogen peroxide lyophilizate.

### 〈Production of NaN₃ glass fiber filter medium〉

A solution of 30 mM NaN₃ - 160 U/ml - 10 mM PIPES (pH 7.4) was prepared. 120 µl of this solution was applied to Tween 20-treated GA100 glass fiber filter paper cut to a disk of 11 mmφ and lyophilized to obtain NaN₃ glass fiber filter paper.

### 〈Production of an analysis apparatus〉

In the same manner as in Embodiment 1, an analysis apparatus of the construction shown in Fig. 11 was produced. That is, a hydrogen peroxide glass fiber filter paper (10 mmφ) was fixed on the lower substrate made of acrylic resin. In the center of a surface of the filter paper was applied a 8 mmφ dialysis membrane (18/32, Sanko Pure Chemical Industries, Ltd.) with an adhesive double coated tape molded to a 1.6 mmφ disk. Then, anti-CRP antibody insolubilized membrane (10 mmφ) was laminated on the dialysis membrane aligning the centers. The electrode was fixed to the lower substrate such that the center of the communication hole and the center of the anti-CRP antibody-insolubilized membrane (10 mmφ) were aligned.

### Particulars of Electrode

| | |
|---|---|
| Carbon working electrode: | 4 to 7 mmφ ring-like electrode |
| Silver counter electrode: | 8 to 10 mmφ ring-like electrode |
| Diameter of communication hole: | 4 mmφ |

A NaN₃ glass fiber filter paper (11 mmφ) to which was applied a 7mmφ disk-shaped liquid non-permeating seal in the central part of the surface such that the surface on which the liquid non-permeating seal was applied faced up, and the center of the liquid non-permeating seal was aligned with the center of the communication hole of the electrode substrate, was laminated. Further, the HRPO-labeled CRP - THEPD glass fiber filter paper (11 mmφ) was laminated on the NaN₃ glass fiber filter paper. Also, a PET film (12 mmφ) provided with flow inlets (central: 3 mmφ, peripheral: 2 mmφ) as an insertion element was dipped in a 0.5% Tween 20 aqueous solution for about 1 hour. Then, the PET film with the flow inlets was dried, and superimposed on the HRPO-labeled CRP - THEPD glass fiber filer paper such that the center the PET film was aligned with the center of the HRPO-labeled CRP - THEPD glass fiber filer paper (11 mmφ). When the PET film provided with the flow inlets was superimposed, the center of the PET provided with the flow inlets was aligned with the center of the liquid sample introduction inlet of the upper cover made of acrylic resin covering the PET film, moreover the PET film was arranged so that the protrusions provided under the upper cover does not clog the flow inlets of the PET film. Finally, the upper cover to which PET film provided with the flow inlets was fixed was overlaid and the four corners thereof were screwed to fabricate an analysis apparatus as shown in Fig. 11.

### 〈Measurement of electric current〉

200 µl of samples obtained by adding each concentration of CRP to fresh whole blood were injected into the sample introduction inlet of the analysis apparatus and measurement of electric current was carried out. To the working electrode was applied a voltage of - 150 mV with respect to the counter electrode, before the addition of he sample liquid. Electric current was measured, using an electric current measuring circuit (Mochida Pharmaceutical Co., Ltd.) in terms of electric current values at intervals of 1 second. An average value of electric current values from the time when the sample was added up to 270 to 330 seconds, was considered as the electric current value of the working electrode.

### (Results)

As shown in Fig. 17, there was good response to CRP concentration in whole blood cells.

### (Embodiment 3)

### 〈Production method for an analysis apparatus having a thin layer flow passage〉

In the same manner as in Example 2, an analysis apparatus having a thin layer flow passage was fabricated. That is, a hydrogen peroxide lyophilized glass fiber filter paper GB100R (10 mmφ) was fixed on the lower substrate made of acrylic resin. In the center of a surface of the filter paper was applied a 8 mmφ disk-shaped dialysis membrane (18/32, Sanko Pure Chemical Industries, Ltd.). Further, the anti-CRP antibody-insolubilized membrane (10 mmφ) was superimposed on hydrogen peroxide lyophilized glass fiber filter paper (10 mmφ) with aligning the centers. Then the electrode substrate was fixed to the lower substrate such that the center of the communication hole and the center of the anti-CRP antibody-insolubilized membrane (10 mmφ) were aligned.

### Particulars of Electrode

| | |
|---|---|
| Carbon working electrode: | 4 to 7 mmφ ring-like electrode |
| Silver counter electrode: | 8 to 10 mmφ ring-like electrode |
| Diameter of communication hole: | 4 mmφ |

An NaN₃ lyophilized glass fiber filter paper (11 mmφ) to which was applied a 7mmφ disk-shaped liquid non-permeating seal in the central part of the surface was laminated such that the surface on which the liquid non-permeating seal was applied was up and the center of the liquid non-permeating seal was aligned with the center of the communication hole of the electrode. Further, the HRPO-labeled CRP - THEPD lyophilized glass fiber filter paper (11 mmφ) was laminated on the NaN₃ lyophilized glass fiber filter paper with its center being aligned. An upper cover made of acrylic resin having eight radial protrusions for forming space constituting a flow passage securing member was put and further the four corners of it were screwed to fabricate an analysis apparatus having the construction as shown in Fig. 1, which has a flow passage securing member constructed by the space formed between the upper cover and the NaN₃ lyophilized glass fiber filter paper.

### 〈Measurement of current〉

Samples obtained by adding each concentration of CRP to 200 µl of fresh whole blood were injected into the sample introduction inlet of the above-mentioned analysis apparatus having the construction as shown in Fig. 1, and measurement of current was carried out. To the working electrode was applied a voltage of -150 mV with respect to the counter electrode just before the addition of the sample liquid. Current was measured using a current measuring circuit (Mochida Pharmaceutical Co., Ltd.) in terms of current values at intervals of 1 second. An average value of current values up to 360 to 420 seconds from the time when the sample was added was set up as a current value of the working electrode.

### (Results)

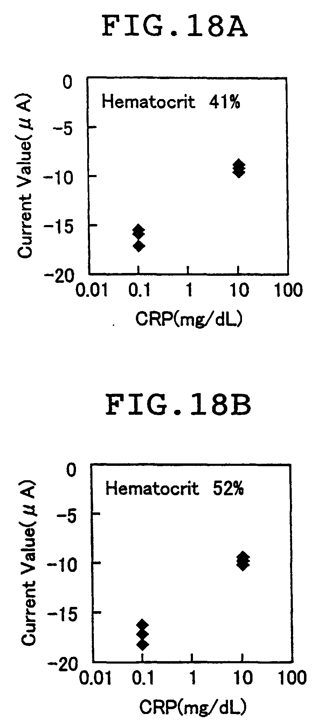

Although whole blood specimens having hematocrit values of 41% and 52%, respectively, were used for analysis, the influence of hematocrit values was suppressed. AS shown in Fig. 18, there was no difference in precision of current values between specimens having different hematocrit values.

### (Embodiment 4)

An analysis apparatus having the construction as shown in Fig. 1 was fabricated. That is, a hydrogen peroxide lyophilized glass fiber filter paper GB100R (10 mmφ) was fixed on the lower substrate made of acrylic resin. In the center of a surface of the filter paper was applied an 8 mmφ disk-shaped dialysis membrane (18/32, Sanko Pure Chemical Industries, Ltd.). Further, the anti-CRP antibody-insolubilized membrane (10 mmφ) was superimposed on hydrogen peroxide lyophilized glass fiber filter paper (10 mmφ) with aligning the centers. Then the electrode substrate was fixed to the lower substrate such that the center of the communication hole and the center of the anti-CRP antibody-insolubilized membrane (10 mmφ) were aligned.

### Particulars of Electrode

| | |
|---|---|
| Carbon working electrode: | 4 to 7 mmφ ring-like electrode |
| Silver counter electrode: | 8 to 10 mmφ ring-like electrode |
| Diameter of communication hole: | 4 mmφ |

An NaN_{*3*} lyophilized glass fiber filter paper (11 mmφ) to which was applied a 7mmφ disk-shaped liquid non-permeating seal in the central part of the surface was laminated such that the surface on which the liquid non-permeating seal was applied was up and the center of the liquid non-permeating seal was aligned with the center of the communication hole of the electrode. Further, the HRPO-labeled CRP - THEPD lyophilized glass fiber filter paper (11 mmφ) was laminated on the NaN₃ lyophilized glass fiber filter paper with its center being aligned. An upper cover shown in Fig. 2 having protrusions of 0.5 mm in height (No. 1 in Table 1), an upper cover shown in Fig. 2 having protrusions of 1.0 mm in height (No. 2 in Table 1), an upper cover shown in Fig. 4 having protrusions of 0.5 mm in height (No. 3 in Table 1), and upper cover shown in Fig. 5 having protrusions of 0.5 mm in height (No. 4 in Table 1), respectively, were put and the four corners of the cover were screwed to fabricate four kinds of analysis apparatuses having flow passage securing member constructed by the space formed between the upper cover and the NaN₃ lyophilized glass fiber filter paper.

### 〈Measurement of current〉

200 µl of a sample obtained by adding each concentration of CRP to fresh whole blood was injected into respective sample introduction inlets of the four analysis apparatuses fabricated as described above and measurement of current was carried out. To the working electrode was applied a voltage of -150 mV with respect to the counter electrode just before the addition of the sample liquid. Current was measured using a current measuring circuit (Mochida Pharmaceutical Co., Ltd.) in terms of current values at intervals of 1 second. An average value of current values up to 360 to 420 seconds from the time when the sample was added was set up as a current value of the working electrode.

### (Results)

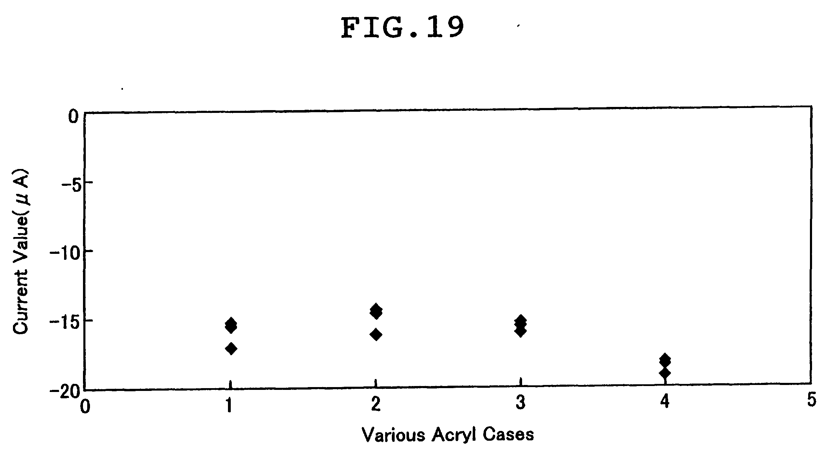

As shown in Table 1 and Fig. 19, the four analysis apparatuses each exhibited good whole blood cell specimens properties. Comparing the upper covers with each other, CV % of current value was the smallest when the upper cover of the form shown in Fig. 4 having cylindrical protrusions of 0.5 mm in height was used.

**Table 1**

| Number | Height of protrusion of upper cover (mm) | Working electrode | |
|---|---|---|---|
| | | Concentration of CRP added 0 mg/dl Current value (µA) | Average SD CV (%) |
| 1 | 0.5 | -17.05 | -15.93 |
| | | -15.45 | 0.9700 |
| | | -15.3 | 6.1 |
| 2 | 1 | -14.4 | -15.12 |
| | | -16.1 | 0.8808 |
| | | -14.85 | 5.8 |
| 3 | 0.5 | -15.6 | -15.62 |
| | | -16.05 | 0.4252 |
| | | -15.2 | 2.7 |
| 4 | 0.5 | -18.35 | -18.60 |
| | | -18.15 | 0.6144 |
| | | -19.3 | 3.3 |

### (Embodiment 5)

### 〈Production of rabbit polyclonal anti-CRP antibody-insolubilized membrane〉

4.0 mg/ml of rabbit polyclonal anti-CRP antibody RC3 (Mochida Pharmaceutical Co., Ltd.) solution was prepared using PBS. A 10 mmφ mixed cellulose porous membrane (Millipore, Inc.) was dipped in the antibody solution for one night for infiltration to insolubilize the antibody. After washing the antibody-insolubilized membrane, it was dipped in a 1-% casein (Wako Pure Chemical Industries, Ltd.)/PBS solution to carry out blocking. Then, the membrane was washed and dried under reduced pressure to obtain an antibody-insolubilized membrane.

An analysis apparatus having the construction as shown in Fig. 1 was fabricated in the same manner as in Example 4. That is, a hydrogen peroxide lyophilized glass fiber filter paper GB100R (100 mmφ) was fixed on the lower substrate made of acrylic resin. In the center of a surface of the filter paper was applied an 8 mmφ disk-shaped dialysis membrane (18/32, Sanko Pure Chemical Industries, Ltd.). Further, the anti-CRP antibody-insolubilized membrane (10 mmφ) was superimposed on the hydrogen peroxide lyophilized glass fiber filter paper (10 mmφ) with aligning the centers. Then, the electrode substrate was fixed to the lower substrate such that the center of the communication hole and the center of the anti-CRP antibody-insolubilized membrane (10 mmφ) were aligned.

### Particulars of Electrode

| | |
|---|---|
| Carbon working electrode: | 4 to 6 mmφ ring-like electrode |
| Silver counter electrode: | 9 to 10 mmφ ring-like electrode |
| Diameter of communication hole: | 4 mmφ, |

An NaN₃ lyophilized glass fiber filter paper (11 mmφ) to which a 7mmφ disk-shaped liquid non-permeating seal was applied in the central part of the surface was laminated such that the surface on which the liquid non-permeating seal was applied was up and the center of the liquid non-permeating seal was aligned with the center of the communication hole of the electrode. Further, the HRPO-labeled CRP - THEPD lyophilized glass fiber filter paper (11 mmφ) was laminated on the NaN₃ lyophilized glass fiber filter paper with its center being aligned. Then, an upper cover shown in Fig. 4 having protrusions of 0.5 mm in height (No. 1 in Table 2), an upper cover shown in Fig. 6 having protrusions of 0.7 mm in height (Nos. 3 and 4 in Table 2), an upper cover shown in Fig. 6 having protrusions of 0.5 mm in height (No. 2 in Table 2), and upper covers shown in Fig. 6 having protrusions of 1.0 mm in height (Nos. 5, 6, and 7 in Table 2), respectively, were put and the four corners of the cover were screwed to fabricate four kinds of analysis apparatuses having flow passage securing member constructed by the space formed between the upper cover and the NaN₃ lyophilized glass fiber filter paper.

### 〈Measurement of current〉

200 µl or 180 µl of a sample obtained by adding each concentration of CRP to 1 ml of fresh whole blood was injected into respective sample introduction inlets of the four analysis apparatuses fabricated as described above and measurement of current was carried out. To the working electrode was applied a voltage of -150 mV with respect to the counter electrode just before the addition of the sample liquid. Current was measured using a current measuring circuit (Mochida Pharmaceutical Co., Ltd.) in terms of current values at intervals of 1 second. An average value of current values up to 360 to 420 seconds from the time when the sample was added was set up as a current value of the working electrode.

### (Results)

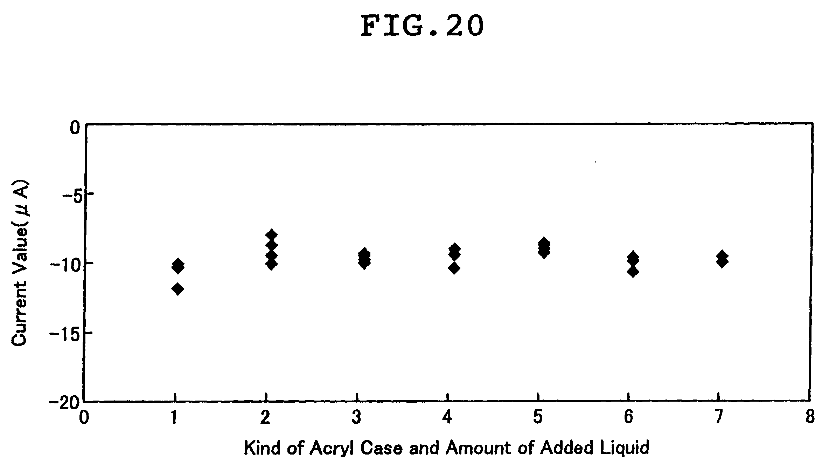

As shown in Fig. 20, the four analysis apparatuses each exhibited good whole blood cell specimens properties. Comparing the upper covers with each other, CV % of current value was the smallest when the upper cover of the form shown in Fig. 6 having cylindrical protrusions of 1.0 mm in height was used and the amount of liquid was set to 180 µl (No. 7 in Table 2).

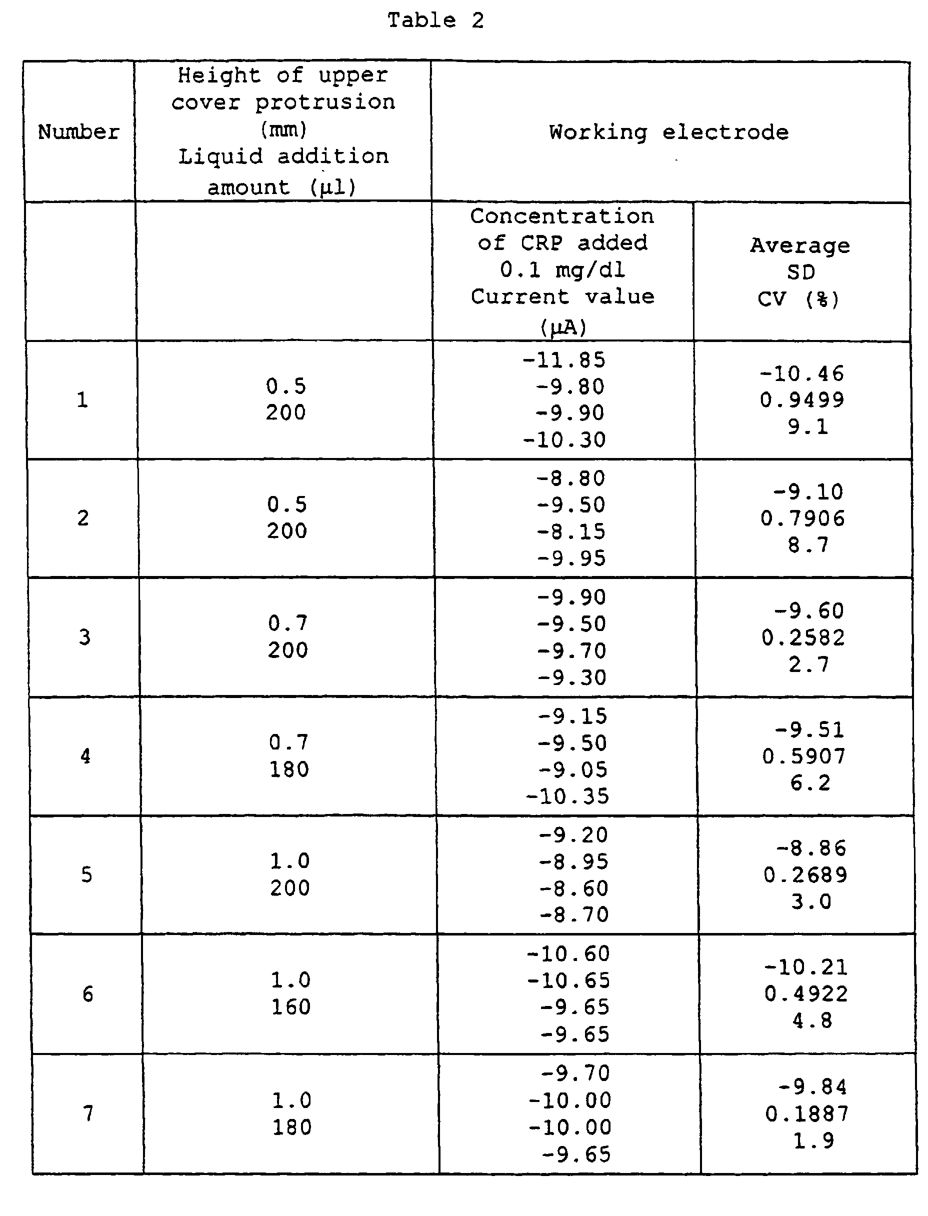

### (Embodiment 6)

### 〈Production of an analysis apparatus having a thin layer flow passage〉

In the same manner as in Example 4, an analysis apparatus of the construction shown in Fig. 1 was fabricated. That is, a hydrogen peroxide glass fiber filter paper (10 mmφ) was fixed on the lower substrate made of acrylic resin. In the center of a surface of the filter paper was attached to a 8 mmφ dialysis membrane (18/32, Sanko Pure Chemical Industries, Ltd.). Then, anti-CRP antibody-insolubilized membrane (10 mmφ) was laminated on the dialysis membrane with aligning the centers. The electrode was fixed to the lower substrate such that the center of the communication hole and the center of the anti-CRP antibody-insolubilized membrane (10 mmφ) were aligned.

### Particulars of Electrode

| | |
|---|---|
| Carbon working electrode: | 4 to 6 mmφ ring-like electrode |
| Silver counter electrode: | 9 to 10 mmφ ring-like electrode |
| Diameter of communication hole: | 4 mmφ |

An NaN₃ glass fiber filter paper (11 mmφ) to which was applied a 7mmφ disk-shaped liquid non-permeating seal in the central part of the surface was laminated such that the surface on which the liquid non-permeating seal was applied was up and the center of the liquid non-permeating seal was aligned with the center of the communication hole of the electrode substrate. Further, the HRPO-labeled CRP - THEPD glass fiber filter paper (11 mmφ) was laminated on the NaN₃ glass fiber filter paper. Then, the HRPO-labeled CRP - THEPD lyophilized glass fiber filter paper (11 mmφ) was further laminated on the NaN₃ lyophilized glass fiber filter paper with aligning the centers. Next, an upper covers shown in Fig. 6 having protrusions of 1.0 mm in height was put and the four corners thereof were screwed to fabricate an analysis apparatus, which has a flow passage securing member constructed by the space formed between the upper cover and the NaN₃ lyophilized glass fiber filter paper.

### 〈Measurement of current〉

180 µl of the samples obtained by adding each concentration of CRP to fresh whole blood was injected into the sample introduction inlet of the analysis apparatus fabricated as described above and measurement of current was carried out. To the working electrode was applied a voltage of -150 mV with respect to the counter electrode just before the addition of the sample liquid. Current was measured using a current measuring circuit (Mochida Pharmaceutical Co., Ltd.) in terms of current values at intervals of 1 second. An average value of current values up to 420 to 480 seconds from the time when the sample was added was set up as a current value of the working electrode.

### (Results)

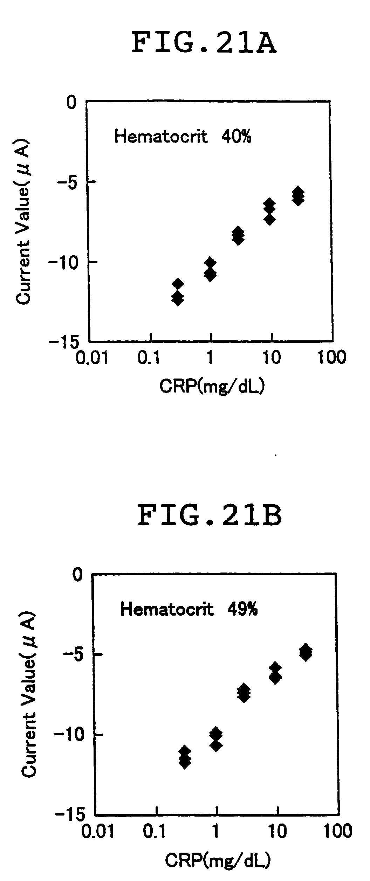

Whole blood specimens having hematocrit values of 40% and 49%, respectively, were used for analysis. With the present analysis apparatus, no influence of hematocrit values was observed as shown in Fig. 21. Also, with respect to the precision of current values, the present analysis apparatus showed no difference between specimens having different hematocrit values.

### (Embodiment 7)

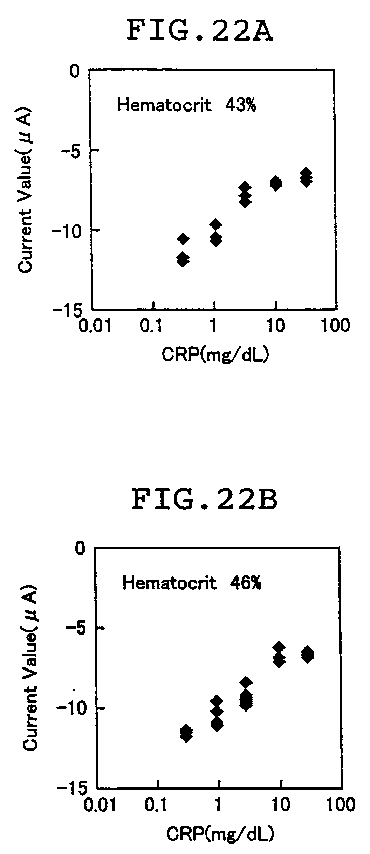

Analysis was performed using whole blood specimens having hematocrit values of 43% and 46%, respectively, in the same manner as in Example 4 except that as the upper cover, use was made of the one having the construction including a plurality of cylindrical protrusions 14a, 14b, 14c, ... surrounded by a circular frame 72 as shown in Fig. 7. As a result, the analysis apparatus showed no influence by hematocrit values as shown in Fig. 22. Also, with respect to the precision of current values, the present analysis apparatus showed no difference between specimens having different hematocrit values.

### (Embodiment 8)

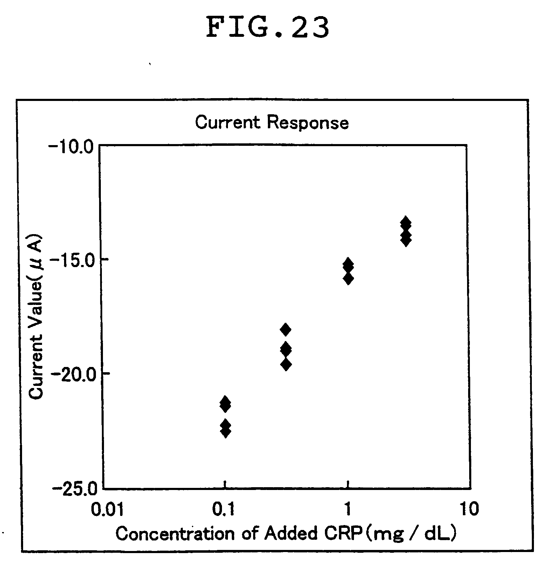

Analysis was performed using whole blood specimens having various hematocrit values, respectively, in the same manner as in Example 4 except that as the upper cover, use was made of the one having the construction including a plurality of cylindrical protrusions 14a, 14b, 14c, ... surrounded by a circular frame 72 as shown in Fig. 7 and that the order of laminating the NaN₃ lyophilized glass fiber filter paper and HRPO-labeled CRP - THEPD lyophilized glass fiber filter paper was reversed. As a result, the analysis apparatus also showed good response to current as shown in Fig. 23 and was not influenced by hematocrit.

### Industrial Applicability

The analyzer for liquid samples of the present invention provides an analysis apparatus for a liquid sample, in which upon analysis of liquid component in a liquid sample possibly containing a solid component, the permeation of the liquid component in the liquid sample in a filter member provided for preliminarily capturing the solid component is not prevented by the captured solid component, so that the degree of accuracy in the analysis of the object substance in the liquid component in an analysis member downstream of the filter member can be improved, the solid component can be efficiently captured on the filter member to achieve quick analysis, and the whole apparatus including the filter member can be downsized.

Further, the specific binding analysis method of the present invention can accurately and efficiently measure an object substance in a liquid sample by its specific binding reaction using the above analyzer.

Furthermore, the specific binding analysis apparatus of the present invention can accurately and efficiently analyze and measure an object substance in a liquid sample by its specific binding reaction using the above specific binding analysis method.

## Claims

1. An analyzer for liquid samples, comprising a liquid sample introduction member, a filter member for capturing a solid component of a liquid sample introduced from the liquid sample introduction member, and an analysis member for analyzing an object substance in the liquid sample passed through the filter, wherein a clearance-carrying flow passage securing member with carrying a clearance is formed between a member, which is provided above the filter member, and the filter member so that the permeation of a liquid component in the liquid sample is not prevented by the solid component captured by and accumulated on the filter member.

2. The analyzer for liquid samples as claimed in claim 1, wherein the flow passage securing member is constituted by a clearance formed between a depression formed on a lower surface of the member formed on a body of the apparatus arranged above the filter member and an upper surface of the filter member.

3. The analyzer for liquid samples as claimed in claim 1, wherein the flow passage securing member is constituted by an insertion member having a liquid passage, inserted between the member arranged above the filter member and the filter member.

4. The analyzer for liquid samples as claimed in any of claims 1 to 3, wherein a liquid non-permeating element having bored with at least one liquid flow inlet is arranged between the flow passage securing member and the filter member.

5. The analyzer for liquid samples as claimed in any of claims 1 to 4, wherein a reagent component comprising an enzyme inhibitor, a blood coagulation inhibitor, or a blood agglutination promoter is arranged at at least one of the liquid sample introduction member, the flow passage securing member, and the filter member.

6. The analyzer for liquid samples as claimed in any of claims 1 to 5, wherein the solid component contained in the liquid sample is a blood cell component.

7. A specific binding analysis method that measures an object substance in a liquid sample based on its specific binding reaction using the analyzer for liquid samples as claimed in any of claims 1 to 4, comprising the steps of: flowing a signal substance generator that participates in a specific binding reaction and generates a signal substance and the liquid sample in a predetermined flow passage in a predetermined direction, generating specific binding reaction of the object substance, forming a distribution of the signal substance generator in the flow passage corresponding to a concentration of the object substance in the liquid sample utilizing the specific binding reaction, generating a signal substance by the signal substance generator distributed in the flow passage, and detecting the generated signal substance by a detection means.

8. A specific binding analysis apparatus for use in the specific binding analysis method as claimed in claim 7, the apparatus comprising a liquid sample introduction member, a flow passage securing member, a filter member, and an analysis member, wherein the analysis member has a flow passage for liquid samples arranged so as to be connected to the filter member and a detection member that detects a distribution of a signal substance generator corresponding to an amount of the object substance in the liquid sample formed in the flow passage by a specific binding reaction of the object substance in the liquid sample with a specific binding substance that specifically binds to the object substance as a signal intensity corresponding to mass transfer by diffusion of the signal substance generated by the signal substance generator.

9. The specific binding analysis apparatus as claimed in claim 8, wherein the detection member is an electrode that detects an electrochemical signal.

10. The specific binding analysis apparatus as claimed in claim 8 or 9, wherein the reagent component comprising an enzyme inhibitor, a blood coagulation inhibitor, or a blood cell agglutination promoter is arranged at at least one of the liquid sample introduction member, the flow passage securing member, and the filter member.

11. The liquid sample analysis apparatus as claimed in any of claims 8 to 10, wherein the solid component contained in the liquid sample is a blood cell component.
